(19) Europäisches Patentamt
European Patent Office
Office européen des brevets

(11) Veröffentlichungsnummer: **0 382 113 B1**

(12) **EUROPÄISCHE PATENTSCHRIFT**

(45) Veröffentlichungstag der Patentschrift: **21.09.94**

(51) Int. Cl.5: **C12P 41/00**, C12P 13/04, C07F 9/32

(21) Anmeldenummer: **90102068.5**

(22) Anmeldetag: **02.02.90**

# (54) Verfahren zur enzymatischen Trennung von 2-Amino-4-methylphosphinobuttersäurederivaten.

(30) Priorität: **06.02.89 DE 3903446**

(43) Veröffentlichungstag der Anmeldung:
**16.08.90 Patentblatt 90/33**

(45) Bekanntmachung des Hinweises auf die Patenterteilung:
**21.09.94 Patentblatt 94/38**

(84) Benannte Vertragsstaaten:
**AT BE CH DE DK ES FR GB IT LI NL SE**

(56) Entgegenhaltungen:
**EP-A- 0 018 415**
**EP-A- 0 054 897**
**EP-A- 0 238 954**
**EP-A- 0 292 918**
**EP-A- 0 301 391**
**FR-A- 2 438 054**

(73) Patentinhaber: **Hoechst Schering AgrEvo GmbH**
**Gerichtstrasse 27**
**D-13342 Berlin (DE)**

(72) Erfinder: **Willms, Lothar, Dr.**
**Lindenstrasse 17**
**D-5416 Hillscheid (DE)**
Erfinder: **Fülling, Gerd, Dr.**
**Drosselweg 3**
**D-6230 Frankfurt am Main 80 (DE)**
Erfinder: **Keller, Reinhold, Dr.**
**Kaunweg 8**
**D-6232 Bad Soden am Taunus (DE)**

EP 0 382 113 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus DE-A-2939269 (US-A-4,226,941) und DE-A-2717440 (US-A-4,168,963) ist bekannt, daß die herbizide Wirkung von racemischem Phosphinothricin oder dessen Salzen mit organischen oder anorganischen Basen oder Säuren von der L-2-Amino-4-methylphosphinobuttersäure (im folgenden als L-Phospinothricin oder L-PTC bezeichnet) bzw. deren Salzen ausgeht. Die D-Form ist praktisch inaktiv. Im Gegensatz zum leicht zugänglichen Racemat des Phosphinothricins ließ sich L-PTC bisher nur nach vergleichsweise aufwendigen Verfahren erhalten. Es erschien damit notwendig, ein brauchbares Verfahren zu entwickeln, durch das die L-Form in ökonomischer Weise zugänglich gemacht werden kann.

Es ist bereits bekannt, daß L-PTC durch saure Hydrolyse (JP-A-73-85538) oder durch enzymatischen Abbau (JP-A-74-31890) von L-PTC-alanyl-alanin, einem literaturbekannten, mikrobiell gewonnenen Antibiotikum, erhalten werden kann.

Weiterhin sind Verfahren bekannt, die auf der enzymatischen Racematspaltung chemisch synthetisierter racemischer PTC-Vorstufen basieren. In der DE-A 2939269 wird ein Verfahren beschrieben, bei dem N-Acyl-PTC, insbesondere N-Acetyl-PTC, mit Hilfe von Acylasen gespalten wird, die aus speziell gezüchteten Mikrobenstämmen der Gattung Pseudomonas, Streptomyces oder Aspergillus erhältlich sind. Dabei wird N-Acyl-L-PTC schneller gespalten als N-Acyl-D-PTC. Die verwendeten Acylasen besitzen gemäß den Angaben der DE-A 2939269 kaum oder nur sehr geringe Wirkung gegenüber anderen Substraten als N-Acyl-L-PTC, beispielsweise gegenüber N-Acylderivaten von üblichen L-Aminosäuren.

Umgekehrt versagen laut DE-A-30 48 612 (US-A-4,389,488) in der Regel handelsübliche Acylasen bei dem Versuch, rac-Acyl-PTC zu spalten. In der DE-A-30 48 612 wird diesbezüglich ein günstiger Einzelfall beschrieben, wonach Penicillin-G-Acylase (Pen-G-Acylase) bei Verwendung von Phenacetyl-PTC verbesserte Aktivität und Selektivität aufweist. Dies war auch insofern überraschend, als eine Variation am Aminosäureteil des Substrats im Vergleich zu phenacetylierten einfachen Aminosäuren, wie rac-Phenacetylalanin oder rac-Phenacetylvalin, nach A. Plaskie, J. Antibiotics 31, 783 (1978) in der Regel einen starken Abfall der Aktivität oder Selektivität von Pen-G-Acylase für die Deacylierung des L-Isomeren erwarten ließ.

Aus der DE-A 29 27 534 (US-A-4,389,489) oder DE-A 22 15 853 (US-A-3,813,317) sind Racematspaltungen von natürlichen und unnatürlichen Aminosäuren, insbesondere arylsubstituierten Aminosäuren, via Carboxylesterspaltung mit Hilfe von proteolytischen Enzymen bekannt, wobei das L-Enantiomer wiederum enzymatisch bevorzugt umgesetzt wird. Es fällt jedoch auf, daß in keiner der erwähnten Veröffentlichungen auf eine Möglichkeit der Spaltung von Aminosäuren mit einem phosphorhaltigen Rest hingewiesen wird. Dies ist wohl darauf zurückzuführen, daß P(V)-haltige Verbindungen, insbesondere Derivate der allgemeinen Formel R'R''P(O)OR''' aufgrund ihrer sterischen Anordnung den Übergangszustand eines enzymatisch hydrolysierten Carboxylesters simulieren (siehe M. Dixon, E. Webb in "Enzymes" e. Edition, Longmans, Grenn & Co LTD, London 1964, S. 346-352) und demzufolge auf hydrolytisch aktive Enzyme einen desaktivierenden Einfluß ausüben können. Tatsächlich wurde auch in der DE-A 30 48 612 beschrieben, daß proteolytische Enzyme mit Esteraseaktivität, welche bei herkömmlichen D,L-Aminosäuren hohe Aktivität und Selektivität aufweisen, bei D,L-PTC-Estern keine oder nur eine stark verminderte Aktivität aufweisen.

Aufgrund des genannten Standes der Technik war es nicht vorherzusehen, daß mit einfach zugänglichen, zweifach oder dreifach geschützten PTC-Derivaten, die am Phosphinsäureteil des PTC modifiziert werden, eine effektive enzymatische Racematspaltung durchgeführt werden könnte.

Gegenstand der Erfindung ist ein Verfahren zur enzymatischen Trennung von PTC-Derivaten, dadurch gekennzeichnet, daß man in Gemisch von D- und L-PTC-Derivaten der allgemeinen Formel (I)

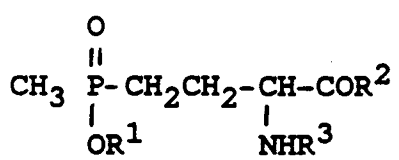

(I),

in welcher

a)

$R^1$ für unverzweigtes oder verzweigtes $C_1$-$C_{20}$-Alkyl, das unsubstituiert oder durch ein oder mehrere Halogenreste, wie Fluor, Chlor, Brom oder Jod, oder ein- oder mehrfach durch $C_1$-$C_8$-Alkoxy substituiert ist, oder für $C_3$-$C_8$-Cycloalkyl, das durch ein oder mehrere Gruppen aus $C_1$-$C_4$-Alkyl, $C_1$-$C_4$-Alkoxy und Halogen substituiert sein kann, oder für $C_3$-$C_{10}$-Alkenyl, $C_3$-$C_{10}$-Alkinyl oder Benzyl steht,

$R^2$ für Hydroxy, unverzweigtes oder verzweigtes $C_1$-$C_{20}$-Alkoxy, das unsubstituiert oder durch ein oder mehrere Halogenresten, wie Fluor, Chlor, Brom und Jod, oder ein- oder mehrfach durch $C_1$-$C_8$-Alkoxy substituiert ist, Amino oder ($C_1$-$C_{20}$-Alkyl)-amino steht und

$R^3$ für Formyl, unverzweigtes oder verzweigtes ($C_1$-$C_{20}$-Alkyl)-carbonyl, das unsubstituiert oder im Alkylteil durch ein oder mehrere Reste aus Hydroxy, Halogen, Phenyl, welches durch bis zu 3 Reste aus der Gruppe $C_1$-$C_{12}$-Alkyl, $C_1$-$C_{12}$-Alkoxy, Halogen, Nitro und $CF_3$ substituiert sein kann, $C_1$-$C_4$-Alkoxy und $C_1$-$C_4$-Alkylthio substituiert ist, für Benzoyl oder Benzoyl, das durch 1 bis 3 Reste aus der Gruppe $C_1$-$C_{12}$-Alkyl, $C_1$-$C_{12}$-Alkoxy, Halogen, Nitro und $CF_3$ substituiert ist, steht oder

b)

$R^1$ wie unter a) definiert ist,

$R^2$ wie unter a) definiert, jedoch kein Hydroxy ist und

$R^3$ wie unter a) definiert ist oder für eine andere für Aminogruppen übliche Schutzgruppe, insbesondere ausgewählt aus unverzweigtem oder verzweigtem ($C_1$-$C_{20}$-Alkoxy)-carbonyl und $C_1$-$C_{20}$-Alkylsulfonyl, die jeweils unsubstituiert oder im Alkyl-Teil durch ein oder mehrere Reste aus der Gruppe Hydroxy, Halogen, $C_1$-$C_4$-Alkoxy, $C_1$-$C_4$-Alkylthio, Phenyl, Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert sind, und Phenylsulfonyl, das durch bis zu drei Resten aus $C_1$-$C_{12}$-Alkyl, $C_1$-$C_{12}$-Alkoxy, Halogen, Nitro und $CF_3$ substituiert sein kann, steht,

in wäßrigem oder wäßrig-organischem Medium mit einem hydrolytisch aktiven Enzym behandelt, wobei im Fall a) vorzugsweise ein N-Acyl-Gruppen spaltendes Enzym (Acylase) und im Fall b) vorzugweise ein proteolytisches oder esteraseaktives Enzym verwendet wird.

Von besonderem Interesse ist ein erfindungsgemäßes Verfahren, dadurch gekennzeichnet, daß man ein Gemisch von D- und L-PTC-Derivaten der genannten Formel (I) verwendet, in welcher

a)

$R^1$ für unverzweigtes oder verzweigtes $C_1$-$C_{10}$-Alkyl oder $C_1$-$C_{10}$-Alkyl, das durch Halogen, wie Fluor und Chlor, oder durch $C_1$-$C_4$-Alkoxy substituiert ist, oder für $C_5$-$C_6$-Cycloalkyl steht,

$R^2$ für Hydroxy, unverzweigtes oder verzweigtes $C_1$-$C_8$-Alkoxy oder $C_1$-$C_8$-Alkoxy, das ein- oder mehrfach durch Halogen, wie Fluor und Chlor, oder durch $C_1$-$C_4$-Alkoxy substituiert ist, oder für Amino oder ($C_1$-$C_{10}$)-Alkylamino steht und

$R^3$ für Formyl, unverzweigtes oder verzweigtes ($C_1$-$C_{10}$-Alkyl)-carbonyl, das unsubstituiert oder im Alkylteil durch ein oder zwei Reste aus der Gruppe Hydroxy, Halogen, Phenyl, einem Phenylrest, der durch 1 bis 3 Reste aus $C_1$-$C_4$-Alkyl, $C_1$-$C_4$-Alkoxy und Halogen substituiert ist, $C_1$-$C_4$-Alkoxy und $C_1$-$C_4$-Alkylthio substituiert ist, oder für Benzoyl oder Benzoyl, das durch 1 bis 3 Reste aus $C_1$-$C_4$-Alkyl, $C_1$-$C_4$-Alkoxy und Halogen substituiert ist, steht oder

b)

$R^1$ wie unter a) definiert ist,

$R^2$ wie unter a) definiert, jedoch kein Hydroxy ist und

$R^3$ wie unter a) definiert ist oder für ($C_1$-$C_{10}$-Alkoxy)-carbonyl, das unsubstituiert ist oder durch ein Hydroxy, Halogen, Methoxy, Ethoxy, Phenyl oder einen Phenylrest, der ein bis drei Substituenten aus der Gruppe $C_1$-$C_4$-Alkyl, $C_1$-$C_4$-Alkoxy und Halogen trägt, substituiert ist, steht.

Bevorzugt ist ein erfindungsgemäßes Verfahren, dadurch gekennzeichnet, daß man ein Gemisch von D- und L-PTC-Derivaten der genannten Formel (I) verwendet, in welcher

a)

$R^1$ für unverzweigtes oder verzweigtes $C_1$-$C_6$-Alkyl oder für Cyclohexyl steht,

$R^2$ für Hydroxy, unverzweigtes oder verzweigtes $C_1$-$C_6$-Alkoxy, vorzugsweise Methoxy oder Ethoxy, oder Amino steht und

$R^3$ für ($C_1$-$C_4$-Alkyl)-carbonyl, vorzugsweise Acetyl, oder ($C_1$-$C_4$-Alkyl)-carbonyl, das durch Phenyl oder durch ein- bis dreifach substituiertes Phenyl, dessen 1 bis 3 Substituenten aus $C_1$-$C_4$-Alkyl, $C_1$-$C_4$-Alkoxy und Halogen ausgewählt sind, substituiert ist, vorzugsweise Phenacetyl, oder für Benzoyl steht oder

b)

$R^1$ für unverzweigtes oder verzweigtes $C_1$-$C_6$-Alkyl oder für Cyclohexyl steht,

$R^2$ für unverzweigtes oder verzweigtes $C_1$-$C_6$-Alkoxy, oder Amino steht und

$R^3$ wie unter a) definiert ist oder für unverzweigtes oder verzweigtes ($C_1$-$C_4$-Alkoxy)-carbonyl, vorzugsweise tert-Butyl-oxycarbonyl, oder Benzyloxycarbonyl, das im Phenylring noch durch bis zu drei Reste aus der Gruppe $C_1$-$C_4$-Alkyl, $C_1$-$C_4$-Alkoxy und Halogen substituiert sein kann, steht.

$C_1$-$C_6$-Alkyl bedeutet insbesondere Methyl, Ethyl, 1-Propyl oder 2-Propyl, n-, i-, tert- oder 2-Butyl, 3-Methyl-but-2-yl, n-, i-, tert-, 2- oder 3-Pentyl, n-Hexyl oder ein stereoisomeres Hexyl.

$C_1$-$C_6$-Alkoxy bedeutet insbesondere ($C_1$-$C_6$-Alkyl)-oxy, wobei der Alkylrest dabei die vorstehend genannte Bedeutung hat.

Wenn nicht näher definiert bedeutet Halogen die Reste Fluor, Chlor, Brom und Jod, vorzugsweise Fluor und Chlor, insbesondere Chlor.

Die erfindungsgemäße Spaltung läßt sich verwirklichen durch N-Acylspaltung der D,L-PTC-Derivate der Formel (I), in der $R^1$, $R^2$ und $R^3$ die unter a) genannten Bedeutungen haben, oder durch hydrolytische enzymatische Carboxylesterspaltung oder Carbonsäureamidspaltung der entsprechenden D,L-PTC-Derivate der Formel (I) in der $R^1$, $R^2$ und $R^3$ die unter b) genannten Bedeutungen haben.

Bei der N-Acylspaltung an der acylierten $\alpha$-Aminogruppe fällt das entsprechende L-PTC-Derivat mit $R^3$ = H an, das sich in an sich üblicher Weise aus der wäßrigen Lösung des Reaktionsgemisches isolieren läßt, z.B. durch extraktive Abtrennung des nicht umgesetzten D-PTC-Derivats und der abgespaltenen Säure der Formel $R^3$-OH bei einem pH-Wert im sauren Bereich mit Hilfe eines organischen Lösungsmittels, wobei das L-PTC-Derivat in der wäßrigen Lösung als Ammoniumsalz verbleibt und anschließend durch Einengen der wäßrigen Lösung zur Trockne isoliert werden kann. Weiterhin ist eine Trennung durch Kristallisation, Destillation, Chromatographie u.a. denkbar. Alternativ kann das Substanzgemisch auch einer alkalischer Hydrolyse, z.B. bei einem pH von 12 oder höher bei Raumtemperatur, unterworfen werden. Die optischen Antipoden des Phosphinothricins liegen dann als freies L-PTC (mit $R^3$ = H) bzw. als N-Acyl-D-PTC vor und lassen sich wie beschrieben (s. die erwähnten DE-A 29 39 269 und DE-A 30 48 612) reinigen.

Für das erfindungsgemäße Verfahren unter N-Acylspaltung kommen Acylasen in Frage, die im wäßrigen oder wäßrig-organischen Medium ausreichend hydrolytisch aktiv sind. Solche Enzyme können in Vorversuchen leicht aus der Gruppe der üblichen Acylasen ausgewählt werden.

Geeignete Enzyme für die erfindungsgemäße N-Acylspaltung phosphinsäureestergeschützter Derivate sind beispielsweise Acylase I (EC 3.5.1.14), insbesondere für N-Acetyl-Derivate, und Penicillin-G-Acylase (EC 3.5.1.11), insbesondere für Phenacetylderivate. Penicillin-G-Acylase wird in der Fachliteratur auch mit anderen Namen, wie z.B. Penicillin-G-Amidase oder Penicillin-amidohydrolase bezeichnet.

Alternativ ist eine enzymatische Spaltung der Carbonsäureesterfunktion bzw. Carbonsäureamidfunktion möglich. In diesem Fall kann die geschützte Aminogruppe in $\alpha$-Stellung zur -CO-O- bzw. -CO-NH-Gruppe durch nahezu jede beliebige N-Schutzgruppe geschützt sein, vorzugsweise durch die gängigen N-Schutzgruppen, wie sie in Th. Greene, Protective Groups in Organic Synthesis, John Wiley & Sons, New York 1981, insbesondere auf S. 218 ff, beschrieben sind.

Als Enzyme kommen für letztgenannten Fall insbesondere proteolytische oder esteraseaktive Enzyme in Frage, insbesondere Serin- und Thiol-Proteinasen, vorzugsweise Subtilisin (EC 3.4.4.16, neue Klassifizierung EC 3.4.21.4), $\alpha$-Chymotrypsin (EC 3.4.4.5, neue Klassifizierung EC 3.4.21.1), Papain (EC 3.4.4.10, neue Klassifizierung EC 3.4.22.2), Ficin (EC 3.4.22.3) oder Bromelain (EC 3.4.22.4), wobei die ersten beiden einen Serinrest im aktiven Zentrum der Aminosäurekette haben, während letztere in der Aminosäurekette Cystein als aktives Zentrum aufweisen. Subtilisin ist bevorzugt. Ebenso lassen sich technische Enzymqualitäten einsetzen, wie sie beispielsweise unter den Firmennamen Maxatase® (Hersteller: Gist-Borcades N.V., Delft/Niederlande) und Alcalase® (Hersteller: Novo Industrie AS, Kopenhagen/Dänemark) bekannt sind.

Die Isolierung des enzymatisch hydrolisierten L-PTC-Derivats mit $R^2$ = OH gelingt nach im Prinzip bekannter oder üblicher Weise, wie z.B. durch Kristallisation, Destillation, Säulen- oder Ionenaustauschchromatographie und insbesondere durch schnelle Extraktion, gegebenenfalls bei reduzierter Temperatur von 0 bis 10 °C, des nicht umgesetzten D-PTC-Derivats aus der wäßrigen Phase (bevorzugter pH ist 6-9) mit Hilfe eines geeigneten organischen Lösungsmittels, wobei das aus einer Carboxylesterspaltung bzw. Carbonsäureamidspaltung entstandene L-PTC-Derivat als Carboxylat-Salz ($R^2 = O^-M^+$) in der wäßrigen Phase verbleibt. Dabei ist der pH-Wert so zu wählen, daß noch keine chemische Hydrolyse eingeleitet wird. Nach Abtrennung des D-Derivats erfolgt die chemische Hydrolyse des L-PTC-Derivats analog üblichen Methoden, beispielsweise mit wäßriger verdünnter Mineralsäure bei einer Temperatur von 20 °C bis Siedetemperatur der Lösung bei Reaktionszeiten von 1 bis 24 Std. Als Mineralsäure eignet sich beispielsweise eine Halogenwasserstoffsäure oder Schwefelsäure, insbesondere verdünnte bis konzentrierte Salzsäure. Aber auch organische Säuren sind in einzelnen Fällen gut geeignet. Bei der chemischen Totalhydrolyse fällt neben L-PTC auch die dem Acylrest $R^3$ entsprechende Carbonsäure an, wobei letztere durch Destillation oder Extraktion mit einem geeigneten organischen Lösungsmittel aus der wäßrig-sauren Lösung entfernt werden kann.

Wenn die wäßrige Phase dann zur Trockne eingeengt wird, verbleibt L-PTC als Ammoniumsalz.

Die verbleibenden D-PTC-Derivate lassen sich gegebenenfalls nach weiterer Reinigung durch Extraktionsverfahren, Destillation, Kristallisation oder Chromatographie von Nebenprodukten der enzymatischen

Hydrolyse reinigen und eventuell nach Racemisierung, z.B. auf thermische Weise oder durch Einwirkung einer Base, beispielsweise eines Alkoholats in alkoholischer Lösung, als D,L-Gemisch erneut der enzymatischen Hydrolyse unterwerfen. Diese Racemisierung mit Base verläuft in der Regel besonders substanzschonend.

Die besagten Enzyme können in freier Form oder nach Immobilisierung gemäß dem Fachmann bekannten oder üblichen Verfahren eingesetzt werden. Das jeweilige Substrat wird in wäßrigem Medium gelöst bzw. suspendiert eingesetzt. Konzentrationen von 0,1 % bis zur gesättigten Lösung (letzteres z.B. bei Arbeiten in Suspension) sind möglich. Der Zusatz von wasserlöslichen Lösungsmitteln wie Dimethylsulfoxid oder Methanol ist im Einzelfall ebenso praktizierbar wie das Arbeiten im Zweiphasengebiet unter Zusatz wasserunlöslicher organischer Lösungsmittel.

Die Reaktionstemperatur für die enzymatischen Spaltungen ist in der Regel 10 - 60 °C, vorzugsweise 20 - 50 °C, insbesondere 20 - 40 °C. Das Verfahren kann beispielsweise als Batchverfahren oder kontinuierlich als Säulenverfahren durchgeführt werden.

Die enzymatische Hydrolyse wird vorzugsweise bei einem pH von 5 bis 9, insbesondere pH 6 bis 8,5 durchgeführt, wobei im Einzelfall zwecks Unterdrückung einer unspezifischen Hydrolyse des Carboxylesters - bzw. -amids auch bei pH 5 bis 6 gearbeitet werden kann.

Der Verlauf der Reaktion kann beispielsweise über HPLC anhand der Abnahme des Substrats verfolgt werden. Im Einzelfall, insbesondere bei der Carbonsäureesterspaltung, läßt sich der Reaktionsverlauf auch durch andere einfache Methoden, z.B. durch die Menge an erforderlicher zudosierter Base bis zur pH-Konstanz, ermittelt werden.

Der Gehalt an L-Verbindung im Produkt der enzymatischen Spaltung kann nach erfolgter alkalischer oder saurer Hydrolyse zum L-PTC und anschließender Derivatisierung nach im Prinzip bekannter Weise (D. Aswad, Analytical Biochemistry 137, 405-409 (1984) mit Hilfe von HPLC bestimmt werden.

Die Ausgangsstoffe der allgemeinen Formel I, ausgenommen solche mit $R^2$ = Amino oder Alkylamino, sind bekannt oder lassen sich nach an sich bekannten Verfahren herstellen (vgl. JP-75-7 237; WO 79-1114; JP 73-91019; Meiji Seika Kenkyo Nempo 1981, (20) 33-8). Einzelne Beispiele hierzu sind im experimentellen Teil aufgeführt. Ausgangsstoffe der Formel (I), in der $R^2$ Amino oder Alkylamino ist, sind neu und können über das dem Carboxylamid entsprechende Nitril durch selektive Hydrolyse und gegebenenfalls N-Alkylierung hergestellt werden. Die genannten Nitrile sind beispielsweise nach EP-A-194 521 (US-A-4,692,541) herstellbar. Gegenstand der Erfindung sind deshalb auch diese neuen Verbindungen, einschließlich deren unter die Formel fallenden reinen Enantiomeren und Diastereomeren sowie Gemische der Stereoisomeren.

Die Bezeichnung D bzw. L bei den gegebenenfalls derivatisierten PTC-Derivaten gibt nur die Konfiguration an dem in α-Stellung zur Carbonsäuregruppe befindlichen C-Atom an, das mit der gegebenenfalls geschützten Aminogruppe verbunden ist. Wegen des zusätzlichen Chiralitätszentrums am Phosphoratom in den mit $R^1$ geschützten PTC-Derivaten sind diese D- bzw. L-PTC-Derivate in der Regel keine reinen Enantiomeren, sondern Gemische von Diastereomeren. Für die enzymatische Spaltung ist, wie sich überraschenderweise gezeigt hat, im wesentlichen nur die Konfiguration am erwähnten α-C-Atom von Bedeutung. Nach Hydrolyse des Restes $R^1$ geht das Chiralitätszentrum am Phosphoratom wegen des schnellen Protonenaustauschs zwischen OH und O praktisch verloren.

Das erfindungsgemäße Verfahren zur Trennung von D,L-PTC-Derivaten und zur Herstellung von L-PTC zeichnet sich durch geringen Salzanfalls bei effektiver Trennung von Substrat-und Produktgemisch aus. Das nach der enzymatischen Trennung anfallende D-PTC-Derivat kann, falls nicht erwünscht, leicht und schonend, gegebenenfalls ohne vorherige Isolierung, racemisiert und somit für eine erneute enzymatische Trennung verwendet werden.

Ein besonderer und unerwarteter Vorteil besteht in der Möglichkeit, bei Einsatz der PTC-Derivate mit erfindungemäß estergeschützter P-O-Säurefunktion kommerziell erhältliche Acylasen zur enantioselektiven Spaltung der PTC-Derivate verwenden zu können. Eine Hemmung der hydrolytischen Aktivität durch den Phosphinsäureester oder gar die Nebenreaktion, eine Hydrolyse des Phosphinsäureesters, wird dabei überraschenderweise in der Regel nicht beobachtet. Insbesondere bei der enzymatischen Carboxylesterspaltung wirkt sich der Schutz der Phosphinsäure als Ester positiv auf die Umsatzgeschwindigkeit aus bzw. macht im Einzelfall die Akzeptanz des Substrats durch das Enzym erst möglich.

Darüberhinaus zeigt sich, daß trotz der starken Veränderungen der strukturellen als auch chemischen Eigenschaften eines erfindungsgemäß eingesetzten PTC-Derivats der Formel (I), in der $R^3$ Phenacetyl bedeutet, gegenüber dem nur mit einer N-Phenacetylgruppe geschützten PTC, wie es nach der DE-A-30 48 612 verwendet wird, ebenfalls eine effektive N-Phenacetylspaltung mit Pen-G-Acylase bei zumindest gleicher, z. T. erhöhter Reaktionsgeschwindigkeit beobachtet wird. Wegen der genannten Vorteile bei der Aufarbeitung, ist das erfindungsgemäße Verfahren auch in diesem Fall überlegen.

## A) Ausgangsmaterialien

### Beispiel A1

D,L-(3-Phenylacetamido-3-ethoxycarbonyl-propyl)-methylphosphinsäureethylester (Formel I: $R^1 = C_2H_5$, $R^2 = OC_2H_5$, $R^3 = C_6H_5$-$CH_2CO$):

8,97 g (0,03 Mol) D,L-(3-Phenylacetamido-3-carboxy-propyl)-methyl-phosphinsäure - hergestellt nach JP-55-0025 (1980) oder EP-A-054897 - werden in einem Gemisch aus 10 ml Eisessig und 80 ml Orthoameisensäuretriethylester gelöst und 3 Std. unter Rückfluß erhitzt. Das Reaktionsgemisch wird einrotiert und an einer Kieselgelsäule (Laufmittel $CH_2Cl_2$/Methanol, 9:1) gereinigt. Man erhält 9,7 g (91 % d. Th.) eines farblosen Harzes.
$^1$H-NMR ($CDCl_3$): $\delta$ = 7,4 ($C_6H_5$,s,5H); 6,7 (NH,t,1H); 4,65 (CH,m,1H); 3,55-4,2 (2 x $\underline{CH_2}CH_3$,m,4H); 3,65 ($CH_2$;s,2H); 1,1-2,2 ($PCH_3$,$CH_2CH_2$, 2 x $\underline{CH_3}CH_2$,m,13 H).

### Beispiel A2

D,L-(3-Phenylacetamido-3-n-butoxycarbonyl-propyl)-methylphosphinsäuremethylester (Formel I: $R^1 = CH_3$, $R^2$ = n-O-$C_4H_9$, $R^3 = C_6H_5$-$CH_2CO$-):

a) D,L-(3-Phenylacetamido-3-n-butoxycarbonyl-propyl)-methylphosphinsäure:
50,0 g (0,167 Mol) D,L-(3-Phenylacetamido-3-carboxy-propyl)-methylphosphinsäure werden in 150 ml n-Butanol gelöst und nach Zugabe einer Spatelspitze p-Toluolsulfonsäure 5 Std. am Rückfluß erhitzt. Anschließend wird das Reaktionsgemisch eingeengt und der Rückstand durch Verreiben mit n-Heptan kristallisiert. Man erhält 50 g (84 % d. Th.) farblose Kristalle mit einem Schmelzpunkt von 90 °C.
b) D,L-(3-Phenylacetamido-3-n-butoxycarbonyl-propyl)-methylphosphinsäuremethylester:
10,0 g (0,028 Mol) des unter A2a) erhaltenen Produktes werden analog Beispiel 1 mit einem Gemisch aus 10 ml Eisessig/80 ml Orthoameisensäuretrimethylester umgesetzt. Man erhält 6,0 g (58,2 % d. Th.) eines farblosen Harzes.
$^1$H-NMR ($CDCl_3$): $\delta$ = 7,2 ($C_6H_5$,m,5H); 6,8 (NH,m,1H); 4,65 (CH,m,1H); 4,05 ($\underline{CH_2}CH_2CH_2CH_3$,t,2H); 3,8 ($POCH_3$,dd,3H); 3,7 ($CH_2$,s,2H), 0,8-2,2 ($CH_2\underline{CH_2}\,\underline{CH_2}\,\underline{CH_3}$,$PCH_3$,$CH_2CH_2$,m,14H).

### Beispiel A3

D,L-(3-Phenylacetamido-3-carboxy-propyl)-methylphosphinsäureethylester

a) D,L-(3-Phenylacetamido-3-benzyloxycarbonyl-propyl)-methylphosphinsäure:
29,9 g (0,1 Mol) D,L-(3-Phenylacetamido-3-carboxy)-methylphosphinsäure werden bei Raumtemperatur in 150 ml Methanol suspendiert und mit 19,2 g (0,106 Mol) Tetramethylammoniumhydroxid-Pentahydrat versetzt. Die Lösung wird einrotiert, in 250 ml absolutem DMF gelöst und bei 0 °C mit 19,3 g (0,113 Mol) Benzylbromid versetzt. Nach 18 h Std. Rühren bei Raumtemperatur wird das Reaktionsgemisch in 600 ml Eiswasser eingerührt und mit $CH_2Cl_2$ mehrfach extrahiert; die $CH_2Cl_2$-Extrakte werden anschließend über Natriumsulfat getrocknet und einrotiert und das Rohprodukt im Hochvakuum bei 50 °C von Lösungsmittelresten befreit. Man erhält 28,6 g (73,5 %) eines farblosen Öls, welches ohne weitere Reinigung in der nächsten Stufe eingesetzt wird.
b) D,L-(3-Phenylacetamido-3-benzyloxycarbonyl-propyl)-methylphosphinsäureethylester:
20,0 g (0,05 Mol) D,L-(3-Phenylacetamido-3-benzyloxycarbonylpropyl)-methylphosphinsäure werden mit 50 ml Eisessig und 150 ml Orthoameisensäuretriethylester 3 Std. unter Rückfluß erhitzt. Das Reaktionsgemisch wird einrotiert und durch Chromatographie an Kieselgel (Laufmittel Essigester/Methanol (8:1)) gereinigt. Man erhält 11,9 g (56,7 % d. Th.) an gewünschtem Produkt, das ohne weitere Reinigung in der nächsten Stufe eingesetzt wird.
c) D,L-(3-Phenylacetamido-3-carboxy-propyl)-methylphosphinsäureethylester:
11,0 g (0,026 Mol) D,L-(3-Phenylacetamido-3-benzyloxycarbonyl-propyl)-methylphosphinsäureethylester werden in 250 ml Ethanol gelöst und nach Zugabe von 5 g Palladium auf Aktivkohle (5 %) bei Normaldruck hydriert. Nach 2 Std. wird der Katalysator abfiltriert, das Filtrat einrotiert und den Rückstand mit n-Heptan verrieben. Man erhält 6,2 g (72,9 % d. Th.) an farblosen Kristallen mit Schmp. 112-117 °C.

**Beispiel A4**

D,L-(3-Acetamido-3-methoxycarbonyl-propyl)-methyl-phosphinsäuremethylester:

10 g (0,05 Mol) D,L-(3-Amino-3-carboxy-propyl)-methylphosphinsäure-Ammoniumsalz werden in 40 ml Eisessig suspendiert und nach Zugabe von 50 ml 1,1,1-Trimethoxyethan 3 Std. unter Rückfluß erhitzt. Anschließend wird filtriert, das Filtrat eingeengt und an Kieselgel (Laufmittel $CH_2Cl_2$) chromatographiert. Man erhält 8,75 g (75 % d. Th.) an gewünschtem Produkt in Form eines gelblichen Öls;
$^1$H-NMR ($CDCl_3$): $\delta$ = 7,18 (NH,m,1H); 4,6 (CH,m,1H); 3,75 ($COOCH_3$,s,3H); 3,7 ($POCH_3$,dd,3H), 1,5-2,2 ($CH_2CH_2$,m,4H); 2,05 ($CH_3CO$-,s,3H); 1,45 ($PCH_3$,d,3H).

**Beispiel A5**

D,L-(3-Acetamido-3-ethoxycarbonyl-propyl)-methyl-phosphinsäureethylester:

10 g (0,05 Mol) D,L-(3-Acetamido-3-carboxy-propyl)-methylphosphinsäure-Ammoniumsalz werden analog Beispiel A4, jedoch mit Eisessig/Orthoameisensäuretriethylester umgesetzt. Man erhält 8 g (57,6 % d. Th) an gewünschtem Produkt in Form eines farblosen Öls.
$^1$H-NMR ($CDCl_3$): $\delta$ = 6,95 (NH,m,1H); 4,6 (CH,m,1H); 4,24 ($COO\underline{CH_2}CH_3$,q,3H); 4,08 ($PO\underline{CH_2}CH_3$,m,3H); 2,07 ($CH_3CO$,s,3H); 1,8-2,2 ($Ch_2CH_2$,m,4H); 1,47 ($PCH_3$,d,3H); 1,2-1,45 ($POCH_2\underline{CH_3}$,m,3H).

**Beispiel A6**

D,L-(3-Phenylacetamido-3-aminocarbonyl-propyl)-methylphosphinsäurecyclohexylester

a) D,L-(3-Phenylacetamido-3-cyano-propyl)-methylphosphinsäurecyclohexylester:
28,7 g (0,1 Mol) D,L-3-Acetoxy-3-cyano-propyl)-methylphosphinsäurecyclohexylester (hergestellt analog EP-A-127577) werden bei 20 °C in 1 Std. zu 29,6 ml konz. Ammoniak getropft. Anschließend wird das Reaktionsgemisch mit $CH_2Cl_2$ extrahiert, der $CH_2Cl_2$-Extrakt über Natriumsulfat getrocknet und mit 10,2 g (0,1 Mol) Triethylamin versetzt. Bei 0 °C werden 15,4 g (0,1 Mol) Phenylessigsäurechlorid zugetropft. Nach 18 Std. Rühren bei Raumtemperatur wird mit 50 ml Wasser versetzt, mit 0,5 N Salzsäure ein pH-Wert von 5 eingestellt und mit $CH_2Cl_2$ extrahiert. Das nach dem Eindampfen des $CH_2Cl_2$-Extrakts verbleibende Öl wird durch Chromatographie an Kieselgel (Laufmittel $CH_2Cl_2$) gereinigt. Man erhält 27,5 g (76 % d. Th.) an gewünschtem Produkt;
$^1$H-NMR ($CDCl_3$): $\delta$ = 9,45 (NH,m,1H); 7,3 ($C_6H_5$,s,5H); 4,95 (CH,m,1H); 4,36 (CH,m,1H); 3,6 ($CH_2$,s,2H); 1,2-2,2 ($CH_2CH_2$,$PCH_3$,$C_6H_{10}$,m,17H).
b) D,L-(3-Phenylacetamido-3-aminocarbonyl-propyl)-methylphosphinsäurecyclohexylester:
6 g (0,0165 Mol) D,L-(3-Phenylacetamido-3-cyano-propyl)-methylphosphinsäurecyclohexylester werden in 40 ml Ameisensäure gelöst, anschließend wird bei Raumtemperatur HCl-Gas eingeleitet. Nach 3 Std. wird das Reaktionsgemisch eingeengt, der Rückstand in Methylenchlorid und Wasser gelöst und mit Natriumhydrogencarbonat ein pH-Wert von 5 eingestellt. Anschließend wird mit $CH_2Cl_2$ extrahiert, und die $CH_2Cl_2$-Extrakte werden über $Na_2SO_4$ getrocknet und einrotiert. Das verbleibende Rohprodukt wird durch Chromatographie an Kieselgel (Laufmittel $CH_2Cl_2$/MeOH, 9:1) gereinigt. Man erhält 4,18 g (66,7 % d.Th.) eines hellgelben Öls;
$^1$H-NMR ($CDCl_3$): $\delta$ = 7,2 ($C_6H_5$,s,5H); 7,2 ($CONH_2$,d,2H); 5,6 (NH,s,1H); 4,2-4,8 (2 x CH,m,2H), 3,6 ($CH_2$,s,2H), 1,2-2,3 ($CH_2CH_2$,$PCH_3$,$C_6H_{10}$,m,17H).

## B) Enzymatische enantiodifferenzierende Hydrolysen

### Beispiel B1

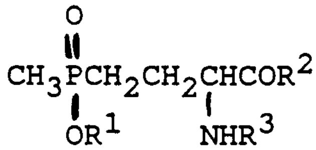

| | $R^1$ | $R^2$ | $R^3$ |
|---|---|---|---|
| (1) | Et | OH | Phac |
| (2) | Et | OH | H |
| PTC | H | OH | H |

1,2 g D,L-(3-Phenylacetamido-3-carboxy-propyl)-methylphosphinsäureethylester (D,L-(1)) werden in 40 ml $H_2O$ aufgenommen und nach Einstellen des pH-Werts auf pH 7,8 mit 0,2 N wäßriger Ammoniaklösung in Gegenwart von 2 ml fixierter Penicillin-G-Acylase (66 units/ml) bei 35 ° C gerührt. Der pH-Wert wird durch Zugabe von 0,2 N wäßriger Ammoniaklösung konstant gehalten. Nach 2 h filtiert man das Enzym ab, stellt mit konzentrierter Salzsäure auf pH 2,5 und extrahiert schnell erschöpfend mit Methylisobutylketon verbleibendes Substrat D-(1) und Phenylessigsäure. Nach Einengen der wäßrigen Phase zur Trockne unter reduziertem Druck erhält man 550 mg L-(3-Amino-3-carboxy-propyl)-methylphosphinsäurethylester-Hydrochlorid (L-(2)), verunreinigt mit $NH_4Cl$; $[\alpha]_D^{22}$ = +8,3 ° (c=5 in $H_2O$),
$^1$H-NMR ($D_2O$) von L-(2): $\delta$ = 3,9-4,3 ($CH_3\underline{CH_2}O$, $\underline{CH}$-$NH_2$, m, 3H); 1,8-2,4 ($CH_2CH_2$, m, 4H); 1,65 (P-$CH_3$, d, 3H); 1,3 ($\underline{CH_3}CH_2O$, t, 3H).

Zur Bestimmung der Selektivität der enzymatischen Spaltung wird das L-(2)-Hydrochlorid zunächst in 20 ml konzentrierter Salzsäure 6 h unter Rückfluß erhitzt und zur Trockne eingeengt, d.h. chemisch hydrolysiert. Eine 10 mg-Probe wird dann in 10 ml einer Pufferlösung bei pH 10 gelöst, mit 134 $\mu$l einer Lösung von 1 g BOC-Cystein in Ethanol sowie 67 $\mu$l einer Lösung von 333 mg o-Phthaldialdehyd in 5 ml Ethanol versetzt und nach 10 min über HPLC (Säule: LiChrosorb RP-18; Laufmittel: 50 mMol Phosphatpuffer/Methanol/Tetrahydrofuran 71/28/1) analysiert; $R_t$ (L-PTC) = 6,0 min; $R_t$ (D-PTC) = 6,6 min. Der Anteil an reinem L-(2)-Hydrochlorid nach der enzymatischen Spaltung ergibt sich somit indirekt über das L-PTC zu mindestens 94 %.

### Beispiel B2

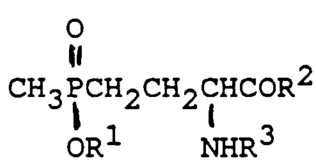

| | $R^1$ | $R^2$ | $R^3$ |
|---|---|---|---|
| (3) | Et | OEt | Phac |
| (1) | Et | OH | Phac |

200 mg D,L-(3-Phenylacetamido-3-ethoxycarbonyl-propyl)-methylphosphinsäureethylester (D,L-(3)) werden in 40 ml $H_2O$ suspendiert und mit 0,2 N wäßriger Ammoniaklösung auf pH 8 eingestellt. Nach Zugabe von 10 mg Subtilisin läßt man 2 h bei 35 °C reagieren. Aus der Lösung wird mit Methylisobutylketon erschöpfend D-(3) extrahiert. Die wäßrige Phase wird mit konzentrierter Salzsäure auf pH 2 gestellt und ebenfalls erschöpfend mit Methylisobutylketon extrahiert. Nach Einengen der wäßrigen Phase zur Trockne erhält man 75 mg L-(3-Phenylacetamido-3-carboxy-propyl)-methylphosphinsäureethylester (L-(1)) mit einem Drehwert von $[\alpha]_D^{22}$ = +25 ° (4 % in $CHCl_3$); $^1$H-NMR ($D_2O$): $\delta$ = 7,3 (Phenyl, 5H); 4,6-4,3 ($\underline{CH}$-NH, m, 1H); 4,3-3,7 ($CH_3\underline{CH_2}O$, m, 2H); 3,65 ($\underline{CH_2}C_6H_5$, s, 2H); 2,4-1,5 ($CH_2CH_2$, m, 4H); 1,5 (P-$\underline{CH_3}$, d, 3H); 1,25 ($\underline{CH_3}CH_2O$, t, 3H).
L-(1) wird in konzentrierter Salzsäure aufgenommen für 6 h unter Rückfluß erhitzt und anschließend zur Trockne eingeengt. Der Anteil an L-PTC wird, wie im Beispiel B1 beschrieben, über HPLC bestimmt: Anteil an L-Verbindung ist demgemäß mindestens 92 %.

**Beispiel B3**

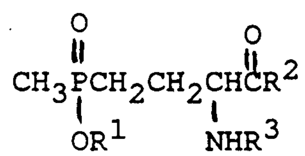

| | $R^1$ | $R^2$ | $R^3$ |
|---|---|---|---|
| (3) | Et | OEt | Phac |
| (4) | Et | OEt | H |

2 g D,L-(3-Phenylacetamido-3-ethoxycarbonyl-propyl)-methylphosphinsäureethylester (D,L-(3)) werden in 40 ml $H_2O$ suspendiert und nach Einstellen des pH-Werts auf pH 8 in Gegenwart von 2 ml fixierter Penicillin-G-Amidase (132 units) bei 35 °C gerührt. Der pH-Wert wird durch Zugabe von 0,2 N wäßriger Ammoniaklösung konstant gehalten. Nach 2 Std. filtriert man das Enzym ab, stellt die Reaktionslösung mit konzentrierter Salzsäure auf pH 2 und extrahiert erschöpfend mit Methylisobutylketon nicht umgesetztes Substrat D-(3) und Phenylessigsäure. Die wäßrige Phase wird im Vakuum zur Trockne eingeengt, wobei 729 mg mit $NH_4Cl$ verunreinigtes L-(3-Amino-3-ethoxycarbonyl)-methyl-phosphinsäureethylester (L-(4)) mit einem Drehwert von $[\alpha]_D^{22}$ = +15° (c=5 in $H_2O$) erhalten werden;
$^1$H-NMR ($D_2O$): $\delta$ = 4,5-3,75 (2 x $CH_3\underline{CH_2}O$, $CHNH_2$, m, 5H); 2,4-1,75 ($CH_2CH_2$, m, 4H); 1,6 (P-$\underline{CH_3}$) d, 3H); 1,3+1,33 (2 x $\underline{CH_3}CH_2O$, t, 6H).
L-(4) wird, wie in Beispiel B1 beschrieben, in L-PTC überführt und analysiert; Anteil an reinem L-(4) ist danach 96 %.

Das extrahierte, enzymatisch nicht umgesetzte Substrat D-(3) wird in Methylisobutylketon aufgenommen, einmal mit 1N NaOH gewaschen und säulenchromatographisch ($CH_2Cl_2$:MeOH 10:1) gereinigt.
Ausbeute: 700 mg D-(3)
$[\alpha]_D^{22}$ = -9° ($CHCl_3$)

**Beispiel B4**

1,2 g (3,8 mmol) D,L-(3-Phenylacetamido-3-methoxycarbonyl-propyl)-methylphosphinsäuremethylester werden in 50 ml 0,01 M wäßrigem Kaliumphosphatpuffer bei pH 7 mit 1,2 g fixierter Penicillin-G-Amidase (240 units) umgesetzt. Nach 24 Std. filtriert man vom Enzym ab, stellt auf pH 3 und extrahiert erschöpfend mit Methylisobutylketon. Die wäßrige Phase wird zur Trockne eingeengt, anschließend durch Zugabe von konzentrierter Salzsäure auf pH 1 eingestellt und für 6 Std. unter Rückfluß zum Sieden erhitzt. Nach Einengen bis zur Trockne erhält man 420 mg L-Phosphinothricin; Anteil L-PTC, bestimmt über HPLC entsprechend Beispiel B1, ist 93 %.

**Beispiel B5**

700 mg (2,95 mmol) D,L-(3-Acetamido-3-methoxycarbonyl-propyl)-methylphosphinsäuremethylester werden in 25 ml Wasser aufgenommen und nach Zugabe von 700 mg Acylase I sowie 0,37 ml einer 0,1 M $CoCl_2$-Lösung bei Raumtemperatur gerührt. Nach 19 h wird auf pH 12 bis 13 gestellt und 24 h bei Raumtemperatur gerührt. Die Lösung wird zur Trockne eingeengt und anschließend durch Ionenaustauschchromatographie an einem sauren Ionenaustauscher gereingt (Entwicklung mit $H_2O$). Die PTC-haltigen Fraktionen (Ninhydrin-Test, HPLC-Kontrolle) werden vereinigt und zur Trockne eingeengt. Man erhält 250 mg eines braunen Pulvers, das L-PTC mit mehr als 95 % Anteil (Bestimmung über HPLC analog Beispiel B1) enthält.

**Beispiel B6**

500 mg optisch reines D-(3-Phenylacetamido-3-ethoxycarbonyl-propyl)-methylphosphinsäureethylester (D-(3)), hervorgegangen aus einer enzymatischen Spaltung wie unter Beispiel B2 beschrieben, werden in 15 ml $H_2O$ aufgenommen und nach Einstellen auf pH 8 mit 2N $NH_4OH$ mit 1 ml Penicillin-G-Acylase (66 u) bei 35 °C gerührt. Bei Verfolgung der Reaktion über HPLC (Säule: LiChrosorb RP-8, Merck Hibar; Laufmittel: 1 g TBAHS + 10 g $KH_2PO_4$ in 1 l $H_2O$; pH 2,1 durch $H_3PO_4$ einstellen + 1 l Methanol, UV-Detektor 206 nm) läßt sich nach 48 h keine Phenylessigsäure nachweisen. Demzufolge kann die Pen-G-Acylase nur L-(3), nicht aber D-(3) spalten.

**Beispiel B7**

500 mg D,L-(3-Phenylacetamido-3-aminocarbonyl-propyl)methyl-phosphinsäurecyclohexylester werden in 20 ml 0,5 M Kaliumphosphatpuffer, pH 8, aufgenommen und nach Zugabe von 2 ml (130 u) fixierter Pen-G-Acylase bei 35 °C gerührt. Der pH-Wert wird durch kontinuierliches Zutitrieren von 0,2 N $NH_3$-Lösung konstant gehalten. Nach 18 Std. filtriert man vom Enzym ab und extrahiert das Filtrat erschöpfend mit Methylenchlorid. Die wäßrige Phase wird mit konzentrierter Salzsäure auf pH 2 gestellt, mit Methylenchlorid Phenylessigsäure extrahiert und die wäßrige Phase zur Trockne eingeengt. Der Rückstand wird in 20 ml konzentrierter Salzsäure aufgenommen, für 7 Std. unter Rückfluß zum Sieden erhitzt und erneut zur Trockne eingeengt.
Die Gehaltsbestimmung an L-PTC erfolgt wie unter Beispiel B1 beschrieben über HPLC; Anteil L-PTC: 94 %.

**Beispiel B8**

Racemisierung

100 mg D-(3-Phenylacetamido-3-ethoxycarbonylpropyl)methylphosphinsäureethylester (D-(3)) mit einem Drehwert von $[\alpha]_D^{20}$ = -9 ° ($CHCl_3$) werden in 12 ml EtOH gelöst und mit 2 mg Natriumethanolat versetzt. Nach 5 h wird die Lösung zur Trockne eingeengt. Der Drehwert des Produkts beträgt $[\alpha]^{22}$ = 0 ° ($CHCl_3$)

**Patentansprüche**
**Patentansprüche für folgende Vertragsstaaten : AT, BE, CH, DE, DK, FR, GB, IT, LI, NL, SE**

1. Verfahren zur enzymatischen Trennung von PTC-(2-Amino-4-methylphosphinobuttersäure)-Derivaten, dadurch gekennzeichnet, daß man ein Gemisch von D- und L-PTC-Derivaten der allgemeinen Formel (I),

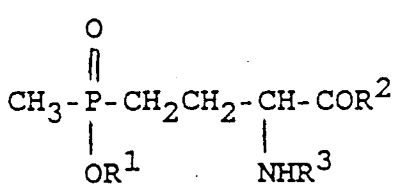

(I),

in welcher

a)

$R^1$ für unverzweigtes oder verzweigtes $C_1$-$C_{20}$-Alkyl, das unsubstituiert oder durch ein oder mehrere Halogenreste oder ein- oder mehrfach durch $C_1$-$C_8$-Alkoxysubstituiert ist, oder für $C_3$-$C_8$-Cycloalkyl, das durch ein oder mehrere Gruppen aus $C_1$-$C_4$-Alkyl, $C_1$-$C_4$-Alkoxy und Halogen substituiert sein kann, oder für $C_3$-$C_{10}$-Alkenyl, $C_3$-$C_{10}$-Alkinyl oder Benzyl, steht,

$R^2$ für Hydroxy, unverzweigtes oder verzweigtes $C_1$-$C_{20}$-Alkoxy, das unsubstituiert oder durch ein oder mehrere Halogenreste oder ein- oder mehrfach durch $C_1$-$C_8$-Alkoxy substituiert ist, Amino oder ($C_1$-$C_{20}$-Alkyl)-amino steht und

$R^3$ für Formyl, unverzweigtes oder verzweigtes ($C_1$-$C_{20}$-Alkyl)-carbonyl, das unsubstituiert oder im Alkylteil durch ein oder mehrere Reste aus Hydroxy, Halogen, Phenyl, welches durch bis zu 3 Reste aus der Gruppe $C_1$-$C_{12}$-Alkyl, $C_1$-$C_{12}$-Alkoxy, Halogen, Nitro und $CF_3$ substituiert sein kann, $C_1$-$C_4$-Alkoxy und $C_1$-$C_4$-Alkylthio substituiert ist, für Benzoyl oder Benzoyl, das durch 1 bis 3 Reste aus der Gruppe $C_1$-$C_{12}$-Alkyl, $C_1$-$C_{12}$-Alkoxy, Halogen, Nitro und $CF_3$ substituiert ist, steht oder

b)

$R^1$ wie unter a) definiert ist,

$R^2$ wie unter a) definiert, jedoch kein Hydroxy ist und

$R^3$ wie unter a) definiert ist oder für eine andere für Aminogruppen übliche Schutzgruppe steht,

in wäßrigem oder wäßrig-organischem Medium mit einem hydrolytisch aktiven Enzym behandelt.

**2.** Verfahren nach Anspruch 1, dadurch gekennzeichnet, daß im Fall a) ein N-Acyl-Gruppen abspaltendes Enzym (Acylase) und im Fall b) ein proteolytisches oder esteraseaktives Enzym als hydrolytisch aktives Enzym verwendet wird.

**3.** Verfahren nach Anspruch 1 oder 2, dadurch gekennzeichnet, daß $R^2$ kein Hydroxy bedeutet und $R^3$ wie in Anspruch 1 unter a) definiert ist oder für eine andere übliche N-Schutzgruppe, ausgewählt aus unverzweigtem oder verzweigtem ($C_1$-$C_{20}$-Alkoxy)-carbonyl und $C_1$-$C_{20}$-Alkylsulfonyl, die jeweils unsubstituiert oder im Alkyl-Teil durch ein oder mehrere Reste aus der Gruppe Hydroxy, Halogen, $C_1$-$C_4$-Alkoxy, $C_1$-$C_4$-Alkylthio, Phenyl, Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert sind, und Phenylsulfonyl, das durch bis zu drei Reste aus $C_1$-$C_{12}$-Alkyl, $C_1$-$C_{12}$-Alkoxy, Halogen, Nitro und $CF_3$ substituiert sein kann, steht.

**4.** Verfahren nach Anspruch 1 oder 2, dadurch gekennzeichnet, daß man ein Gemisch von D- und L-PTC-(2-Amino-4-methylphosphinobuttersäure)-Derivaten der genannten Formel (I) nach Anspruch 1 verwendet, in welcher

a)

$R^1$ für unverzweigtes oder verzweigtes $C_1$-$C_{10}$-Alkyl oder $C_1$-$C_{10}$-Alkyl, das durch Halogen oder durch $C_1$-$C_4$-Alkoxy substituiert ist, oder für $C_5$-$C_6$-Cycloalkyl steht,

$R^2$ für Hydroxy, unverzweigtes oder verzweigtes $C_1$-$C_8$-Alkoxy oder $C_1$-$C_8$-Alkoxy, das ein- oder mehrfach durch Halogen oder durch $C_1$-$C_4$-Alkoxy substituiert ist, oder für Amino oder $C_1$-$C_{10}$-Alkylamino steht, und

$R^3$ für Formyl, unverzweigtes oder verzweigtes ($C_1$-$C_{10}$-Alkyl)-carbonyl, das unsubstituiert oder im Alkylteil durch ein oder zwei Reste aus der Gruppe Hydroxy, Halogen, Phenyl, einen Phenylrest, der durch 1 bis 3 Reste aus $C_1$-$C_4$-Alkyl, $C_1$-$C_4$-Alkoxy und Halogen substituiert ist, $C_1$-$C_4$-Alkoxy und $C_1$-$C_4$-Alkylthio substituiert ist, oder für Benzoyl oder Benzoyl, das durch 1 bis 3 Reste aus $C_1$-$C_4$-Alkyl, $C_1$-$C_4$-Alkoxy und Halogen substituiert ist, steht oder

b)

$R^1$ wie unter a) definiert ist,

$R^2$ wie unter a) definiert, jedoch kein Hydroxy ist und

$R^3$ wie unter a) definiert ist oder für ($C_1$-$C_{10}$-Alkoxy)-carbonyl, das unsubstituiert ist oder durch ein Hydroxy, Halogen, Methoxy, Ethoxy, Phenyl oder einen Phenylrest, der ein bis drei Substituenten aus der Gruppe $C_1$-$C_4$-Alkyl, $C_1$-$C_4$-Alkoxy und Halogen trägt, substituiert ist, steht.

**5.** Verfahren nach Anspruch 1 oder 2, dadurch gekennzeichnet, daß man ein Gemisch von D- und L-PTC-(2-Amino-4-methylphosphinobuttersäure)-Derivaten der Formel (I) nach Anspruch 1 verwendet, in welcher

a)

$R^1$ für unverzweigtes oder verzweigtes $C_1$-$C_6$-Alkyl oder für Cyclohexyl steht,

$R^2$ für Hydroxy, unverzweigtes oder verzweigtes $C_1$-$C_6$-Alkoxy oder Amino steht und

$R^3$ für ($C_1$-$C_4$-Alkyl)-carbonyl oder ($C_1$-$C_4$-Alkyl)-carbonyl, das durch Phenyl oder ein- bis dreifach substituiertes Phenyl, dessen 1 bis 3 Substituenten aus $C_1$-$C_4$-Alkyl, $C_1$-$C_4$-Alkoxy und Halogen ausgewählt sind, substituiert ist, oder für Benzoyl steht oder

b)

$R^1$ für unverzweigtes oder verzweigtes $C_1$-$C_6$-Alkyl oder für Cyclohexyl steht,

$R^2$ für unverzweigtes oder verzweigtes $C_1$-$C_6$-Alkoxy oder Amino steht und

$R^3$ wie unter a) definiert ist oder für unverzweigtes oder verzweigtes ($C_1$-$C_4$-Alkoxy)-carbonyl, vorzugsweise tert-Butyl-oxycarbonyl, oder Benzyloxycarbonyl, das im Phenylring noch durch bis zu drei Reste aus der Gruppe $C_1$-$C_4$-Alkyl, $C_1$-$C_4$-Alkoxy und Halogen substituiert sein kann, steht.

**6.** Verfahren nach Anspruch 1 oder 2, dadurch gekennzeichnet, daß

a)

$R^1$ für Methyl, Ethyl, Propyl, Butyl, Pentyl, Hexyl oder Cyclohexyl,

$R^2$ für Hydroxy, Methoxy, Ethoxy, Propoxy, Butoxy, Pentoxy, Hexoxy oder $NH_2$ und

$R^3$ für Acetyl oder Phenacetyl stehen oder

b)

$R^1$ wie unter a) definiert ist,

$R^2$ wie unter a) definiert ist jedoch kein Hydroxy ist und

$R^3$ wie unter a) definiert ist oder für tert-Butyloxycarbonyl oder Benzyloxycarbonyl steht.

**7.** Verfahren nach einem oder mehreren der Ansprüche 1 bis 6, dadurch gekennzeichnet, daß im Fall

a, Acylase I oder Pen-G-Acylase und im Fall

b, Subtilisin, $\alpha$-Chymotrypsin, Papain, Ficin, Bromelain oder eine technische Enzymqualität als hydrolytisch aktives Enzym eingesetzt wird.

**8.** Verfahren nach einem oder mehreren der Ansprüche 1 bis 7, dadurch gekennzeichnet, daß die enzymatische Spaltung bei einer Temperatur von 20 bis 60 °C, vorzugsweise 20 bis 50 °C durchgeführt wird.

**9.** Verfahren nach einem oder mehreren der Ansprüche 1 bis 8, dadurch gekennzeichnet, daß nach der enzymatischen Spaltung das enzymatisch nicht umgesetzte D-PTC-(2-Amino-4-methylphosphinobuttersäure)-Derivat durch Extraktion mit einem organischen Lösungsmittel entfernt wird.

**10.** Verfahren nach einem oder mehreren der Ansprüche 1 bis 9, dadurch gekennzeichnet, daß das erhaltene L-PTC-(2-Amino-4-methylphosphinobuttersäure)-Derivat einer chemischen Hydrolyse unterworfen und das L-PTC isoliert wird.

**11.** Verfahren nach einem oder mehreren der Ansprüche 1 bis 10, dadurch gekennzeichnet, daß die enzymatische Spaltung bei pH 5 bis 9, insbesondere 6 bis 8,5, durchgeführt wird.

**12.** Verbindungen der nach Anspruch 1 definierten allgemeinen Formel (I), in welcher $R^1$ und $R^3$ nach einem oder mehreren der Ansprüche 1 bis 4 definiert sind und $R^2$ Amino oder $C_1$-$C_{20}$-Alkylamino bedeutet.

**13.** Verbindung nach Anspruch 12, dadurch gekennzeichnet, daß $R^2$ für Amino steht.

**14.** Verbindung nach Anspruch 13, dadurch gekennzeichnet, daß $R^1$ und $R^3$ nach Anspruch 5 oder 6 definiert sind.

**15.** Verbindung nach Anspruch 14, dadurch gekennzeichnet, daß $R^1$ Cyclohexyl und $R^3$ Phenacetyl bedeuten.

**16.** Verfahren zur Herstellung der nach Anspruch 13 definierten Verbindungen, dadurch gekennzeichnet, daß ein Nitril der Formel

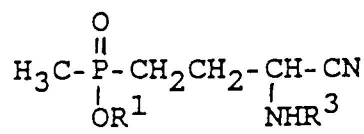

im sauren Medium selektiv an der Cyanogruppe hydrolysiert wird und, sofern gewünscht, das erhaltene Amid nach üblichen Methoden zum ($C_1$-$C_{20}$-Alkyl)amid der Formel (I) alkyliert wird.

**Patentansprüche für folgenden Vertragsstaat : ES**

**1.** Verfahren zur enzymatischen Trennung von PTC-(2-Amino-4-methylphosphinobuttersäure)-Derivaten, dadurch gekennzeichnet, daß man ein Gemisch von D- und L-PTC-Derivaten der allgemeinen Formel (I),

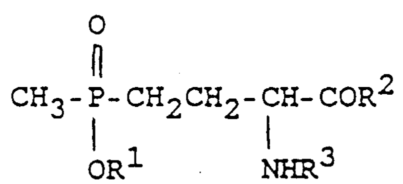

(I),

in welcher

a)

$R^1$ für unverzweigtes oder verzweigtes $C_1$-$C_{20}$-Alkyl, das unsubstituiert oder durch ein oder mehrere Halogenreste oder ein- oder mehrfach durch $C_1$-$C_8$-Alkoxysubstituiert ist, oder für $C_3$-$C_8$-Cycloalkyl, das durch ein oder mehrere Gruppen aus $C_1$-$C_4$-Alkyl, $C_1$-$C_4$-Alkoxy und Halogen substituiert sein kann, oder für $C_3$-$C_{10}$-Alkenyl, $C_3$-$C_{10}$-Alkinyl oder Benzyl, steht,

$R^2$ für Hydroxy, unverzweigtes oder verzweigtes $C_1$-$C_{20}$-Alkoxy, das unsubstituiert oder durch ein oder mehrere Halogenreste oder ein- oder mehrfach durch $C_1$-$C_8$-Alkoxy substituiert ist, Amino oder ($C_1$-$C_{20}$-Alkyl)-amino steht und

$R^3$ für Formyl, unverzweigtes oder verzweigtes ($C_1$-$C_{20}$-Alkyl)-carbonyl, das unsubstituiert oder im Alkylteil durch ein oder mehrere Reste aus Hydroxy, Halogen, Phenyl, welches durch bis zu 3 Reste aus der Gruppe $C_1$-$C_{12}$-Alkyl, $C_1$-$C_{12}$-Alkoxy, Halogen, Nitro und $CF_3$ substituiert sein kann, $C_1$-$C_4$-Alkoxy und $C_1$-$C_4$-Alkylthio substituiert ist, für Benzoyl oder Benzoyl, das durch 1 bis 3 Reste aus der Gruppe $C_1$-$C_{12}$-Alkyl, $C_1$-$C_{12}$-Alkoxy, Halogen, Nitro und $CF_3$ substituiert ist, steht oder

b)

$R^1$ wie unter a) definiert ist,

$R^2$ wie unter a) definiert, jedoch kein Hydroxy ist und

$R^3$ wie unter a) definiert ist oder für eine andere für Aminogruppen übliche Schutzgruppe steht,

in wäßrigem oder wäßrig-organischem Medium mit einem hydrolytisch aktiven Enzym behandelt.

**2.** Verfahren nach Anspruch 1, dadurch gekennzeichnet, daß im Fall a) ein N-Acyl-Gruppen abspaltendes Enzym (Acylase) und im Fall b) ein proteolytisches oder esteraseaktives Enzym als hydrolytisch aktives Enzym verwendet wird.

**3.** Verfahren nach Anspruch 1 oder 2, dadurch gekennzeichnet, daß $R^2$ kein Hydroxy bedeutet und $R^3$ wie in Anspruch 1 unter a) definiert ist oder für eine andere übliche N-Schutzgruppe, ausgewählt aus unverzweigtem oder verzweigtem ($C_1$-$C_{20}$-Alkoxy)-carbonyl und $C_1$-$C_{20}$-Alkylsulfonyl, die jeweils unsubstituiert oder im Alkyl-Teil durch ein oder mehrere Reste aus der Gruppe Hydroxy, Halogen, $C_1$-$C_4$-Alkoxy, $C_1$-$C_4$-Alkylthio, Phenyl, Phenyl, das 1 bis 3 Substituenten aus der Gruppe $C_1$-$C_{12}$-Alkyl, $C_1$-

$C_{12}$-Alkoxy, Halogen, Nitro und $CF_3$ trägt, substituiert sind, und Phenylsulfonyl, das durch bis zu drei Reste aus $C_1$-$C_{12}$-Alkyl, $C_1$-$C_{12}$-Alkoxy, Halogen, Nitro und $CF_3$ substituiert sein kann, steht.

**4.** Verfahren nach Anspruch 1 oder 2, dadurch gekennzeichnet, daß man ein Gemisch von D- und L-PTC-(2-Amino-4-methylphosphinobuttersäure)-Derivaten der genannten Formel (I) nach Anspruch 1 verwendet, in welcher

a)
- $R^1$ für unverzweigtes oder verzweigtes $C_1$-$C_{10}$-Alkyl oder $C_1$-$C_{10}$-Alkyl, das durch Halogen oder durch $C_1$-$C_4$-Alkoxy substituiert ist, oder für $C_5$-$C_6$-Cycloalkyl steht,
- $R^2$ für Hydroxy, unverzweigtes oder verzweigtes $C_1$-$C_8$-Alkoxy oder $C_1$-$C_8$-Alkoxy, das ein- oder mehrfach durch Halogen oder durch $C_1$-$C_4$-Alkoxy substituiert ist, oder für Amino oder $C_1$-$C_{10}$-Alkylamino steht, und
- $R^3$ für Formyl, unverzweigtes oder verzweigtes ($C_1$-$C_{10}$-Alkyl)-carbonyl, das unsubstituiert oder im Alkylteil durch ein oder zwei Reste aus der Gruppe Hydroxy, Halogen, Phenyl, einen Phenylrest, der durch 1 bis 3 Reste aus $C_1$-$C_4$-Alkyl, $C_1$-$C_4$-Alkoxy und Halogen substituiert ist, $C_1$-$C_4$-Alkoxy und $C_1$-$C_4$-Alkylthio substituiert ist, oder für Benzoyl oder Benzoyl, das durch 1 bis 3 Reste aus $C_1$-$C_4$-Alkyl, $C_1$-$C_4$-Alkoxy und Halogen substituiert ist, steht oder

b)
- $R^1$ wie unter a) definiert ist,
- $R^2$ wie unter a) definiert, jedoch kein Hydroxy ist und
- $R^3$ wie unter a) definiert ist oder für ($C_1$-$C_{10}$-Alkoxy)-carbonyl, das unsubstituiert ist oder durch ein Hydroxy, Halogen, Methoxy, Ethoxy, Phenyl oder einen Phenylrest, der ein bis drei Substituenten aus der Gruppe $C_1$-$C_4$-Alkyl, $C_1$-$C_4$-Alkoxy und Halogen trägt, substituiert ist, steht.

**5.** Verfahren nach Anspruch 1 oder 2, dadurch gekennzeichnet, daß man ein Gemisch von D- und L-PTC-(2-Amino-4-methylphosphinobuttersäure)-Derivaten der Formel (I) nach Anspruch 1 verwendet, in welcher

a)
- $R^1$ für unverzweigtes oder verzweigtes $C_1$-$C_6$-Alkyl oder für Cyclohexyl steht,
- $R^2$ für Hydroxy, unverzweigtes oder verzweigtes $C_1$-$C_6$-Alkoxy oder Amino steht und
- $R^3$ für ($C_1$-$C_4$-Alkyl)-carbonyl oder ($C_1$-$C_4$-Alkyl)-carbonyl, das durch Phenyl oder ein- bis dreifach substituiertes Phenyl, dessen 1 bis 3 Substituenten aus $C_1$-$C_4$-Alkyl, $C_1$-$C_4$-Alkoxy und Halogen ausgewählt sind, substituiert ist, oder für Benzoyl steht oder

b)
- $R^1$ für unverzweigtes oder verzweigtes $C_1$-$C_6$-Alkyl oder für Cyclohexyl steht,
- $R^2$ für unverzweigtes oder verzweigtes $C_1$-$C_6$-Alkoxy oder Amino steht und
- $R^3$ wie unter a) definiert ist oder für unverzweigtes oder verzweigtes ($C_1$-$C_4$-Alkoxy)-carbonyl, vorzugsweise tert-Butyl-oxycarbonyl, oder Benzyloxycarbonyl, das im Phenylring noch durch bis zu drei Reste aus der Gruppe $C_1$-$C_4$-Alkyl, $C_1$-$C_4$-Alkoxy und Halogen substituiert sein kann, steht.

**6.** Verfahren nach Anspruch 1 oder 2, dadurch gekennzeichnet, daß

a)
- $R^1$ für Methyl, Ethyl, Propyl, Butyl, Pentyl, Hexyl oder Cyclohexyl,
- $R^2$ für Hydroxy, Methoxy, Ethoxy, Propoxy, Butoxy, Pentoxy, Hexoxy oder $NH_2$ und
- $R^3$ für Acetyl oder Phenacetyl stehen oder

b)
- $R^1$ wie unter a) definiert ist,
- $R^2$ wie unter a) definiert ist jedoch kein Hydroxy ist und
- $R^3$ wie unter a) definiert ist oder für tert-Butyloxycarbonyl oder Benzyloxycarbonyl steht.

**7.** Verfahren nach einem oder mehreren der Ansprüche 1 bis 6, dadurch gekennzeichnet, daß im Fall

a, Acylase I oder Pen-G-Acylase und im Fall

b, Subtilisin, α-Chymotrypsin, Papain, Ficin, Bromelain oder eine technische Enzymqualität als hydrolytisch aktives Enzym eingesetzt wird.

**8.** Verfahren nach einem oder mehreren der Ansprüche 1 bis 7, dadurch gekennzeichnet, daß die enzymatische Spaltung bei einer Temperatur von 20 bis 60 °C, vorzugsweise 20 bis 50 °C durchgeführt wird.

**9.** Verfahren nach einem oder mehreren der Ansprüche 1 bis 8, dadurch gekennzeichnet, daß nach der enzymatischen Spaltung das enzymatisch nicht umgesetzte D-PTC-(2-Amino-4-methylphosphinobuttersäure)-Derivat durch Extraktion mit einem organischen Lösungsmittel entfernt wird.

**10.** Verfahren nach einem oder mehreren der Ansprüche 1 bis 9, dadurch gekennzeichnet, daß das erhaltene L-PTC-(2-Amino-4-methylphosphinobuttersäure)-Derivat einer chemischen Hydrolyse unterworfen und das L-PTC isoliert wird.

**11.** Verfahren nach einem oder mehreren der Ansprüche 1 bis 10, dadurch gekennzeichnet, daß die enzymatische Spaltung bei pH 5 bis 9, insbesondere 6 bis 8,5, durchgeführt wird.

**12.** Verfahren zur Herstellung von Verbindungen der nach Anspruch 1 definierten allgemeinen Formel (I), in welcher $R^1$ und $R^3$ nach einem oder mehreren der Ansprüche 1 bis 4 definiert sind und $R^2$ Amino oder $C_1$-$C_{20}$-Alkylamino bedeutet, dadurch gekennzeichnet, daß ein Nitril der Formel

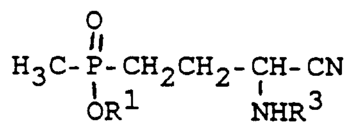

im sauren Medium selektiv an der Cyanogruppe hydrolysiert und, sofern gewünscht, das erhaltene Amid der Formel (I) nach üblichen Methoden zu einem entsprechenden $C_1$-$C_{20}$-Alkylamid der Formel (I) alkyliert wird.

**13.** Verfahren nach Anspruch 12, dadurch gekennzeichnet, daß $R^2$ für Amino steht.

**14.** Verfahren nach Anspruch 13, dadurch gekennzeichnet, daß $R^1$ und $R^3$ nach Anspruch 5 oder 6 definiert sind.

**15.** Verfahren nach Anspruch 14, dadurch gekennzeichnet, daß $R^1$ Cyclohexyl und $R^3$ Phenacetyl bedeuten.

**Claims**

**Claims for the following Contracting States : AT, BE, CH, DE, DK, FR, GB, IT, LI, NL, SE**

**1.** A process for the enzymatic resolution of PTC (2-amino-4-methylphosphinobutyricacid) derivatives, which comprises treating a mixture of D- and L-PTC derivatives of the formula (I)

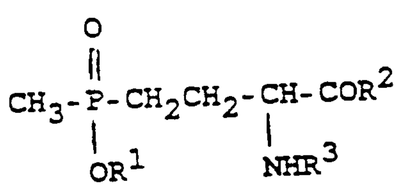

(I),

in which

a)

$R^1$ is unbranched or branched $C_1$-$C_{20}$-alkyl which is unsubstituted or substituted by one or more halogen radicals, or monosubstituted or polysubstituted by $C_1$-$C_8$-alkoxy, or $R^1$ is $C_3$-$C_8$-cycloalkyl which can be substituted by one or more groups from the series comprising $C_1$-$C_4$-alkyl, $C_1$-$C_4$-alkoxy and halogen, or $R^1$ is $C_3$-$C_{10}$-alkenyl, $C_3$-$C_{10}$-alkynyl or benzyl,

$R^2$ is hydroxyl, unbranched or branched $C_1$-$C_{20}$-alkoxy which is unsubstituted or substituted

by one or more halogen radicals, or monosubstituted or polysubstituted by $C_1$-$C_8$-alkoxy, or $R^2$ is amino or ($C_1$-$C_{20}$-alkyl)amino, and

$R^3$ is formyl, unbranched or branched ($C_1$-$C_{20}$-alkyl)carbonyl which is unsubstituted or substituted in the alkyl moiety by one or more radicals from the series comprising hydroxyl, halogen, $C_1$-$C_4$-alkoxy, $C_1$-$C_4$-alkylthio, and phenyl which can be substituted by up to 3 radicals from the group comprising $C_1$-$C_{12}$-alkyl, $C_1$-$C_{12}$-alkoxy, halogen, nitro and $CF_3$, or $R^3$ is benzoyl or benzoyl which is substituted by 1 to 3 radicals from the group comprising $C_1$-$C_{12}$-alkyl, $C_1$-$C_{12}$-alkoxy, halogen, nitro and $CF_3$, or

b)

$R^1$ is as defined under a),

$R^2$ is as defined under a), but is not hydroxyl, and

$R^3$ is as defined under a) or is another protective group customary in the case of amino groups,

with a hydrolytically active enzyme in an aqueous or aqueous-organic medium.

**2.** The process as claimed in claim 1, wherein, in case a), an enzyme is used which detaches N-acyl groups (acylase) and, in case b), a proteolytic or esterase-active enzyme is used as the hydrolytically active enzyme.

**3.** The process as claimed in claim 1 or 2, wherein $R^2$ is not hydroxyl and $R^3$ is as defined in claim 1 under a) or is another customary N-protective group, selected from amongst unbranched or branched ($C_1$-$C_{20}$-alkoxy)carbonyl and $C_1$-$C_{20}$-alkylsulfonyl, each of which is unsubstituted or substituted in the alkyl moiety by one or more radicals from the group comprising hydroxyl, halogen, $C_1$-$C_4$-alkoxy, $C_1$-$C_4$-alkylthio, phenyl or phenyl which has 1 to 3 substituents selected from the group comprising $C_1$-$C_{12}$-alkyl, $C_1$-$C_{12}$-alkoxy, halogen, nitro and $CF_3$, and phenylsulfonyl which can be substituted by up to three radicals from amongst $C_1$-$C_{12}$-alkyl, $C_1$-$C_{12}$-alkoxy, halogen, nitro and $CF_3$.

**4.** The process as claimed in claim 1 or 2, wherein a mixture is used of D- and L-PTC (2-amino-4-methylphosphinobutyric acid) derivatives of the indicated formula (I) as claimed in claim 1, in which formula

a)

$R^1$ is unbranched or branched $C_1$-$C_{10}$-alkyl or $C_1$-$C_{10}$-alkyl which is substituted by halogen or by $C_1$-$C_4$-alkoxy, or $R^1$ is $C_5$-$C_6$-cycloalkyl,

$R^2$ is hydroxyl, unbranched or branched $C_1$-$C_8$-alkoxy or $C_1$-$C_8$-alkoxy which is monosubstituted or polysubstituted by halogen or by $C_1$-$C_4$-alkoxy, or $R^2$ is amino or $C_1$-$C_{10}$-alkylamino, and

$R^3$ is formyl, unbranched or branched ($C_1$-$C_{10}$-alkyl)carbonyl which is unsubstituted or substituted in the alkyl moiety by one or two radicals from the group comprising hydroxyl, halogen, phenyl, $C_1$-$C_4$-alkoxy, $C_1$-$C_4$-alkylthio, a phenyl radical which is substituted by 1 to 3 radicals selected from amongst $C_1$-$C_4$-alkyl, $C_1$-$C_4$-alkoxy and halogen, or $R^3$ is benzoyl or benzoyl which is substituted by 1 to 3 radicals selected from amongst $C_1$-$C_4$-alkyl, $C_1$-$C_4$-alkoxy and halogen, or

b)

$R^1$ is as defined under a),

$R^2$ is as defined under a), but is not hydroxyl, and

$R^3$ is as defined under a) or is ($C_1$-$C_{10}$-alkoxy)carbonyl which is unsubstituted or substituted by hydroxyl, halogen, methoxy, ethoxy, phenyl, or a phenyl radical which carries one to three substituents from the group comprising $C_1$-$C_4$-alkyl, $C_1$-$C_4$-alkoxy and halogen.

**5.** The process as claimed in claim 1 or 2, wherein a mixture is used of D- and L-PTC (2-amino-4-methylphosphinobutyric acid) derivatives of the formula (I) as claimed in claim 1, in which

a)

$R^1$ is unbranched or branched $C_1$-$C_6$-alkyl or is cyclohexyl,

$R^2$ is hydroxyl, unbranched or branched $C_1$-$C_6$-alkoxy, or amino, and

$R^3$ is ($C_1$-$C_4$-alkyl)carbonyl, or ($C_1$-$C_4$-alkyl)carbonyl which is substituted by phenyl or by phenyl which is monosubstituted to trisubstituted and whose 1 to 3 substituents are selected from amongst $C_1$-$C_4$-alkyl, $C_1$-$C_4$-alkoxy and halogen, or $R^3$ is benzoyl, or

b)
$R^1$ is unbranched or branched $C_1$-$C_6$-alkyl or is cyclohexyl,
$R^2$ is unbranched or branched $C_1$-$C_6$-alkoxy, or amino, and
$R^3$ is as defined under a) or is unbranched or branched ($C_1$-$C_4$-alkoxy)carbonyl, preferably tert-butyloxycarbonyl, or is benzyloxycarbonyl which additionally can be substituted in the phenyl ring by up to three radicals from the group comprising $C_1$-$C_4$-alkyl, $C_1$-$C_4$-alkoxy and halogen.

**6.** The process as claimed in claim 1 or 2, wherein
a)
$R^1$ is methyl, ethyl, propyl, butyl, pentyl, hexyl or cyclohexyl,
$R^2$ is hydroxyl, methoxy, ethoxy, propoxy, butoxy, pentoxy, hexoxy or $NH_2$ and
$R^3$ is acetyl or phenacetyl, or
b)
$R^1$ is as defined under a),
$R^2$ is as defined under a) and
$R^3$ is as defined under a), or is tert-butyloxycarbonyl or benzyloxycarbonyl.

**7.** The process as claimed in one or more of claims 1 to 6, wherein, in case
a), acylase I or pen-G-acylase is employed, and, in case
b), subtilisine, $\alpha$-chymotripsin, papain, ficin, bromelain or an industrial enzyme grade is employed as the hydrolytically active enzyme.

**8.** The process as claimed in one or more of claims 1 to 7, wherein the enzymatic cleavage is carried out at a temperature of 20 to 60 °C, preferably 20 to 50 °C.

**9.** The process as claimed in one or more of claims 1 to 8, wherein the enzymatically unreacted D-PTC (2-amino-4-methylphosphinobutyric acid) derivative is removed after the enzymatic cleavage by extraction with an organic solvent.

**10.** The process as claimed in one or more of claims 1 to 9, wherein the L-PTC (2-amino-4-methylphosphinobutyric acid) derivative obtained is subjected to chemical hydrolysis and the L-PTC is isolated.

**11.** The process as claimed in one or more of claims 1 to 10, wherein the enzymatic cleavage is carried out at pH 5 to 9, in particular 6 to 8.5.

**12.** A compound of the formula (I) as defined in claim 1, in which $R^1$ and $R^3$ are defined as claimed in one or more of claims 1 to 4, and $R^2$ is amino or $C_1$-$C_{20}$-alkylamino.

**13.** A compound as claimed in claim 12, wherein $R^2$ is amino.

**14.** A compound as claimed in claim 13, wherein $R^1$ and $R^3$ are as defined in claim 5 or 6.

**15.** A compound as claimed in claim 14, wherein $R^1$ is cyclohexyl and $R^3$ is phenacetyl.

**16.** A process for the preparation of a compound as defined in claim 13, wherein a nitrile of the formula

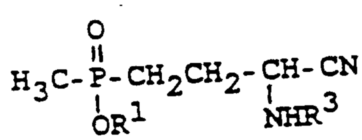

is selectively hydrolyzed on the cyano group in an acid medium, and if desired, the resulting amide is alkylated by customary methods to give the ($C_1$-$C_{20}$-alkyl)amide of the formula (I).

**Claims for the following Contracting State : ES**

1. A process for the enzymatic resolution of PTC (2-amino-4-methylphosphinobutyricacid) derivatives, which comprises treating a mixture of D- and L-PTC derivatives of the formula (I)

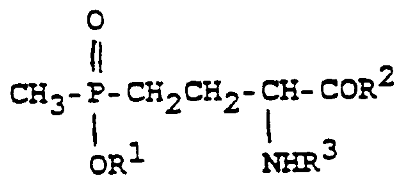

(I),

in which

a)

$R^1$ is unbranched or branched $C_1$-$C_{20}$-alkyl which is unsubstituted or substituted by one or more halogen radicals, or monosubstituted or polysubstituted by $C_1$-$C_8$-alkoxy, or $R^1$ is $C_3$-$C_8$-cycloalkyl which can be substituted by one or more groups from the series comprising $C_1$-$C_4$-alkyl, $C_1$-$C_4$-alkoxy and halogen, or $R^1$ is $C_3$-$C_{10}$-alkenyl, $C_3$-$C_{10}$-alkynyl or benzyl,

$R^2$ is hydroxyl, unbranched or branched $C_1$-$C_{20}$-alkoxy which is unsubstituted or substituted by one or more halogen radicals, or monosubstituted or polysubstituted by $C_1$-$C_8$-alkoxy, or $R^2$ is amino or ($C_1$-$C_{20}$-alkyl)amino, and

$R^3$ is formyl, unbranched or branched ($C_1$-$C_{20}$-alkyl)carbonyl which is unsubstituted or substituted in the alkyl moiety by one or more radicals from the series comprising hydroxyl, halogen, $C_1$-$C_4$-alkoxy, $C_1$-$C_4$-alkylthio, and phenyl which can be substituted by up to 3 radicals from the group comprising $C_1$-$C_{12}$-alkyl, $C_1$-$C_{12}$-alkoxy, halogen, nitro and $CF_3$, or $R^3$ is benzoyl or benzoyl which is substituted by 1 to 3 radicals from the group comprising $C_1$-$C_{12}$-alkyl, $C_1$-$C_{12}$-alkoxy, halogen, nitro and $CF_3$, or

b)

$R^1$ is as defined under a),

$R^2$ is as defined under a), but is not hydroxyl, and

$R^3$ is as defined under a) or is another protective group customary in the case of amino groups,

with a hydrolytically active enzyme in an aqueous or aqueous-organic medium.

2. The process as claimed in claim 1, wherein, in case a), an enzyme is used which detaches N-acyl groups (acylase) and, in case b), a proteolytic or esterase-active enzyme is used as the hydrolytically active enzyme.

3. The process as claimed in claim 1 or 2, wherein $R^2$ is not hydroxyl and $R^3$ is as defined in claim 1 under a) or is another customary N-protective group, selected from amongst unbranched or branched ($C_1$-$C_{20}$-alkoxy) carbonyl and $C_1$-$C_{20}$-alkylsulfonyl, each of which is unsubstituted or substituted in the alkyl moiety by one or more radicals from the group comprising hydroxyl, halogen, $C_1$-$C_4$-alkoxy, $C_1$-$C_4$-alkylthio, phenyl or phenyl which has 1 to 3 substituents selected from the group comprising $C_1$-$C_{12}$-alkyl, $C_1$-$C_{12}$-alkoxy, halogen, nitro and $CF_3$, and phenylsulfonyl which can be substituted by up to three radicals from amongst $C_1$-$C_{12}$-alkyl, $C_1$-$C_{12}$-alkoxy, halogen, nitro and $CF_3$.

4. The process as claimed in claim 1 or 2, wherein a mixture is used of D- and L-PTC (2-amino-4-methylphosphinobutyric acid) derivatives of the indicated formula (I) as claimed in claim 1, in which formula

a)

$R^1$ is unbranched or branched $C_1$-$C_{10}$-alkyl or $C_1$-$C_{10}$-alkyl which is substituted by halogen or by $C_1$-$C_4$-alkoxy, or $R^1$ is $C_5$-$C_6$-cycloalkyl,

$R^2$ is hydroxyl, unbranched or branched $C_1$-$C_8$-alkoxy or $C_1$-$C_8$-alkoxy which is monosubstituted or polysubstituted by halogen or by $C_1$-$C_4$-alkoxy, or $R^2$ is amino or $C_1$-$C_{10}$-alkylamino, and

$R^3$ is formyl, unbranched or branched ($C_1$-$C_{10}$-alkyl)carbonyl which is unsubstituted or substituted in the alkyl moiety by one or two radicals from the group comprising hydroxyl,

halogen, phenyl, $C_1$-$C_4$-alkoxy, $C_1$-$C_4$-alkylthio, a phenyl radical which is substituted by 1 to 3 radicals selected from amongst $C_1$-$C_4$-alkyl, $C_1$-$C_4$-alkoxy and halogen, or $R^3$ is benzoyl or benzoyl which is substituted by 1 to 3 radicals selected from amongst $C_1$-$C_4$-alkyl, $C_1$-$C_4$-alkoxy and halogen, or

b)

$R^1$ is as defined under a),

$R^2$ is as defined under a), but is not hydroxyl, and

$R^3$ is as defined under a) or is ($C_1$-$C_{10}$-alkoxy)carbonyl which is unsubstituted or substituted by hydroxyl, halogen, methoxy, ethoxy, phenyl, or a phenyl radical which carries one to three substituents from the group comprising $C_1$-$C_4$-alkyl, $C_1$-$C_4$-alkoxy and halogen.

**5.** The process as claimed in claim 1 or 2, wherein a mixture is used of D- and L-PTC (2-amino-4-methylphosphinobutyric acid) derivatives of the formula (I) as claimed in claim 1, in which

a)

$R^1$ is unbranched or branched $C_1$-$C_6$-alkyl or is cyclohexyl,

$R^2$ is hydroxyl, unbranched or branched $C_1$-$C_6$-alkoxy, or amino, and

$R^3$ is ($C_1$-$C_4$-alkyl)carbonyl, or ($C_1$-$C_4$-alkyl)carbonyl which is substituted by phenyl or by phenyl which is monosubstituted to trisubstituted and whose 1 to 3 substituents are selected from amongst $C_1$-$C_4$-alkyl, $C_1$-$C_4$-alkoxy and halogen, or $R^3$ is benzoyl, or

b)

$R^1$ is unbranched or branched $C_1$-$C_6$-alkyl or is cyclohexyl,

$R^2$ is unbranched or branched $C_1$-$C_6$-alkoxy, or amino, and

$R^3$ is as defined for a) or is unbranched or branched ($C_1$-$C_4$-alkoxy)carbonyl, preferably tert.-butyloxycarbonyl, or is benzyloxycarbonyl which additionally can be substituted in the phenyl ring by up to three radicals from the group comprising $C_1$-$C_4$-alkyl, $C_1$-$C_4$-alkoxy and halogen.

**6.** The process as claimed in claim 1 or 2, wherein

a)

$R^1$ is methyl, ethyl, propyl, butyl, pentyl, hexyl or cyclohexyl,

$R^2$ is hydroxyl, methoxy, ethoxy, propoxy, butoxy, pentoxy, hexoxy or $NH_2$ and

$R^3$ is acetyl or phenacetyl, or

b)

$R^1$ is as defined under a),

$R^2$ is as defined under a), but is not hydroxyl, and

$R^3$ is as defined under a), or is tert-butyloxycarbonyl or benzyloxycarbonyl.

**7.** The process as claimed in one or more of claims 1 to 6, wherein, in case
a), acylase I or pen-G-acylase is employed, and, in case
b), subtilisine, $\alpha$-chymotripsin, papain, ficin, bromelain, or an industrial enzyme grade is employed as the hydrolytically active enzyme.

**8.** The process as claimed in one or more of claims 1 to 7, wherein the enzymatic cleavage is carried out at a temperature of 20 to 60 °C, preferably 20 to 50 °C.

**9.** The process as claimed in one or more of claims 1 to 8, wherein the enzymatically unreacted D-PTC (2-amino-4-methylphosphinobutyric acid) derivative is removed after the enzymatic cleavage by extraction with an organic solvent.

**10.** The process as claimed in one or more of claims 1 to 9, wherein the L-PTC (2-amino-4-methylphosphinobutyric acid) derivative obtained is subjected to chemical hydrolysis and the L-PTC is isolated.

**11.** The process as claimed in one or more of claims 1 to 10, wherein the enzymatic cleavage is carried out at pH 5 to 9, in particular 6 to 8.5.

**12.** A process for the preparation of a compound of the formula (I) as defined in claim 1, in which $R^1$ and $R^3$ are defined as claimed in one or more of claims 1 to 4, and $R^2$ is amino or $C_1$-$C_{20}$-alkylamino,

wherein a nitrile of the formula

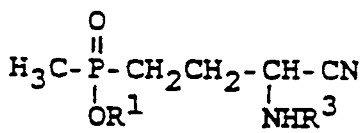

is selectively hydrolyzed on the cyano group in an acid medium, and if desired, the resulting amide is alkylated by customary methods to give the ($C_1$-$C_{20}$-alkyl)amide of the formula (I).

**13.** The process as claimed in claim 12, wherein $R^2$ is amino.

**14.** The process as claimed in claim 13, wherein $R^1$ and $R^3$ are as defined in claim 5 or 6.

**15.** The process as claimed in claim 14, wherein $R^1$ is cyclohexyl and $R^3$ is phenacetyl.

**Revendications**
**Revendications pour les Etats contractants suivants : AT, BE, CH, DE, DK, FR, GB, IT, LI, NL, SE**

**1.** Procédé pour la séparation enzymatique de dérivés de l'acide PTC-(2-amino-4-méthylphospinobutyrique) caractérisé en ce qu'on traite un mélange de dérivés D- et L-PTC de formule générale I :

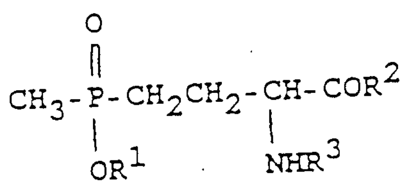

(I),

dans laquelle

a)

$R^1$ représente un alkyle en $C_1$-$C_{20}$ linéaire ou ramifié qui peut être non substitué ou substitué par un ou plusieurs radicaux halogène ou une ou plusieurs fois par un alcoxy en $C_1$-$C_8$, ou représente un cycloalkyle en $C_3$-$C_8$, qui peut être substitué par un ou plusieurs groupes pris parmi les alkyle en $C_1$-$C_4$, alcoxy en $C_1$-$C_4$ et halogène, ou représente les alcènyle en $C_3$-$C_{10}$, alcynyle en $C_3$-$C_{10}$ ou benzyle,

$R^2$ représente les hydroxy, alcoxy en $C_1$-$C_{20}$ linéaire ou ramifié qui est non substitué ou substitué par un ou plusieurs radicaux halogène, une ou plusieurs fois substitué par un alcoxy en $C_1$-$C_8$, ou représente les amino ou (alkyle en $C_1$-$C_{20}$)amino et

$R^3$ représente les formyle, (alkyle en $C_1$-$C_{20}$)carbonyle linéaire ou ramifié, qui est non substitué ou substitué dans la partie alkyle par un ou plusieurs radicaux pris dans le groupe des hydroxy, halogène, phényle, lequel phényle peut être substitué par jusqu'à 3 radicaux pris parmi les groupes alkyle en $C_1$-$C_{12}$, alcoxy en $C_1$-$C_{12}$, halogène, nitro et $CF_3$, ledit (alkyle en $C_1$-$C_{20}$)carbonyle est aussi substitué par alcoxy en $C_1$-$C_4$ et alkylthio en $C_1$-$C_4$, $R^3$ représente aussi les benzoyle ou benzoyle substitué par 1 à 3 radicaux pris dans le groupe comportant les alkyle en $C_1$-$C_{12}$, alcoxy en $C_1$-$C_{12}$, halogène, nitro et $CF_3$,

b)

$R^1$ est défini comme dans a),

$R^2$ est défini comme dans a), mais n'est pas l'hydroxy, et

$R^3$ est défini comme dans a) ou représente un autre groupe protecteur usuel de groupes amino,

en milieu aqueux ou aqueux-organique avec une enzyme à activité hydrolytique.

**2.** Procédé selon la revendication 1, caractérisé en ce qu'on utilise dans le cas a) une enzyme séparant les groupes N-acyle (acylase) et dans le cas b) une enzyme protéolytique ou à activité estérase en tant qu'enzyme hydrolytiquement active.

**3.** Procédé selon la revendication 1 ou 2, caractérisé en ce que $R^2$ ne signifie pas l'hydroxy et $R^3$ est défini comme dans la revendication 1 dans le point a), ou représente un autre groupe N-protecteur usuel, choisi parmi les (alcoxy en $C_1$-$C_{20}$)carbonyle et (alkyle en $C_1$-$C_{20}$)sulfonyle non ramifiés ou ramifiés, qui peuvent être chaque fois non substitués ou substitués dans la partie alkyle par un ou plusieurs radicaux pris dans le groupe comportant les hydroxy, halogène, alcoxy en $C_1$-$C_4$, alkylthio en $C_1$-$C_4$, phényle, phényle portant 1 à 3 substituants pris dans le groupe comportant les alkyle en $C_1$-$C_{12}$, alcoxy en $C_1$-$C_{12}$, halogènes, nitro et $CF_3$, et représente un phénylsulfonyle, qui peut être substitué par jusqu'à trois radicaux pris dans le groupe des alkyle en $C_1$-$C_{12}$, alcoxy en $C_1$-$C_{12}$, halogène, nitro et $CF_3$.

**4.** Procédé selon la revendication 1 ou 2, caractérisé en ce qu'on utilise un mélange de dérivés de l'acide D- et L-PTC-(2-amino-4-méthylphosphinobutyrique) de formule (I) mentionnée selon la revendication 1, dans laquelle

a)

- $R^1$ représente un alkyle en $C_1$-$C_{10}$ linéaire ou ramifié ou un alkyle en $C_1$-$C_{10}$ qui est substitué par des halogène ou alcoxy en $C_1$-$C_4$, ou représente un cycloalkyle en $C_5$-$C_6$,
- $R^2$ représente les hydroxy, alcoxy en $C_1$-$C_8$ linéaire ou ramifié ou alcoxy en $C_1$-$C_8$ qui est une ou plusieurs fois substitué par des halogène ou alcoxy en $C_1$-$C_4$, ou représente les amino ou (alkyle en $C_1$-$C_{10}$)amino, et
- $R^3$ représente les formyle, (alkyle en $C_1$-$C_{10}$)carbonyle linéaire ou ramifié, qui peut être non substitué ou substitué dans la partie alkyle par un ou deux radicaux pris dans le groupe des hydroxy, halogène, phényle, un radical phényle qui est substitué par 1 à 3 radicaux pris parmi les groupes des alkyle en $C_1$-$C_4$, alcoxy en $C_1$-$C_4$ et halogène, et $R^3$ est substitué par les alcoxy en $C_1$-$C_4$ ou (alkyl en $C_1$-$C_4$)-thio ou représente les benzoyle ou benzoyle qui est substitué par 1 à 3 radicaux pris parmi les alkyle en $C_1$-$C_4$, alcoxy en $C_1$-$C_4$, et halogène, ou

b)

- $R^1$ est défini comme dans a),
- $R^2$ est défini comme dans a), mais n'est pas l'hydroxy, et
- $R^3$ est défini comme dans a) ou représente un (alcoxy en $C_1$-$C_{10}$)carbonyle qui est non substitué ou substitué par des hydroxy, halogène, méthoxy, éthoxy, phényle ou un radical phényle qui porte un à trois substituants pris dans le groupe des alkyle en $C_1$-$C_4$, alcoxy en $C_1$-$C_4$ et halogène.

**5.** Procédé selon la revendication 1 ou 2, caractérisé en ce qu'on utilise un mélange de dérivés de l'acide D- et L-PTC-(2-amino-4-méthylphosphinobutyrique) de formule (I) selon la revendication 1, dans laquelle

a)

- $R^1$ représente un alkyle en $C_1$-$C_6$ linéaire ou ramifié ou le cyclohexyle,
- $R^2$ représente les hydroxy, alcoxy en $C_1$-$C_6$ linéaire ou ramifié ou amino, et
- $R^3$ représente un (alkyle en $C_1$-$C_4$)carbonyle ou un (alkyle en $C_1$-$C_4$)carbonyle qui est substitué par un phényle ou un phényle un à trois fois substitué, dont les 1 à 3 substituants sont pris dans le groupe des alkyle en $C_1$-$C_4$, alcoxy en $C_1$-$C_4$ et halogène ou $R^3$ représente le benzoyle ou

b)

- $R^1$ représente un alkyle en $C_1$-$C_6$ linéaire ou ramifié ou le cyclohexyle,
- $R^2$ représente un alcoxy en $C_1$-$C_6$, linéaire ou ramifié, ou un amino, et
- $R^3$ est défini comme dans le point a) ou représente un (alcoxy en $C_1$-$C_4$)-carbonyle linéaire ou ramifié, de préférence le tert-butyl-oxycarbonyle ou le benzyloxycarbonyle qui peut être substitué dans le cycle de phényle encore par un à trois radicaux pris dans le groupe comprenant les alkyle en $C_1$-$C_4$, alcoxy en $C_1$-$C_4$ et halogène.

**6.** Procédé selon la revendication 1 ou 2, caractérisé en ce que

a)

- $R^1$ représente les méthyle, éthyle, propyle, butyle, pentyle, hexyle ou cyclohexyle,
- $R^2$ représente les méthoxy, éthoxy, propoxy, butoxy, pentoxy, hexoxy ou $NH_2$, et
- $R^3$ représente les acétyle ou phénacétyle ou

b)

- $R^1$ est défini comme dans a),
- $R^2$ est défini comme dans a), mais n'est pas l'hydroxy et
- $R^3$ est défini comme dans a) ou représente les tert-butyl-oxycarbonyle ou benzyloxycarbonyle.

**7.** Procédé selon une ou plusieurs des revendications 1 à 6, caractérisé en ce qu'on utilise dans le cas
a) l'acylase I ou la pen-G-acylase, et dans le cas
b) les subtilisine, $\alpha$-chymotrypsine, papaine, ficine, bromélaine ou une autre enzyme de qualité industrielle en tant qu'enzyme à activité hydrolytique.

**8.** Procédé selon une ou plusieurs des revendications 1 à 7, caractérisé en ce qu'on effectue le clivage enzymatique à une température de 20 à 60 ° C, de préférence de 20 à 50 ° C.

**9.** Procédé selon une ou plusieurs des revendications 1 à 8, caractérisé en ce que, après le clivage enzymatique, les dérivés de l'acide D-PTC-(2-amino-4-méthylphosphinobutyrique) n'ayant pas réagi par voie enzymatique, sont éliminés par extraction avec un solvant organique.

**10.** Procédé selon une ou plusieurs des revendications 1 à 9, caractérisé en ce qu'on traite le dérivé obtenu de l'acide L-PTC(2-amino-4-méthylphosphinobutyrique) par une hydrolyse chimique et on isole le L-PTC.

**11.** Procédé selon une ou plusieurs des revendications 1 à 10, caractérisé en ce qu'on effectue le clivage enzymatique à un pH de 5 à 9, plus particulièrement de 6 à 8,5.

**12.** Composés de formule générale I définie selon la revendication 1, dans laquelle $R^1$ et $R^3$ sont définis selon une ou plusieurs des revendications 1 à 4 et R2 signifie un amino ou un (alkyle en $C_1$-$C_{20}$)amino.

**13.** Composé selon la revendication 12, caractérisé en ce que $R^2$ représente un amino.

**14.** Composé selon la revendication 13, caractérisé en ce que $R^1$ et $R^3$ sont définis selon la revendication 5 ou 6.

**15.** Composé selon la revendication 14, caractérisé en ce que $R^1$ représente le cyclohexyle et $R^3$ le phénacétyle.

**16.** Procédé pour la préparation des composés définis selon la revendication 13, caractérisé en ce que, un un nitrile de formule

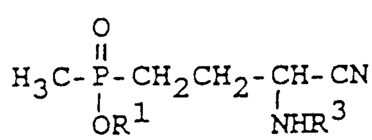

est hydrolysé en milieu acide sélectivement sur le groupe cyano et, si on le désire, l'amide de formule (I) obtenu est alkylé selon des méthodes usuelles en (alkyl en $C_1$-$C_{20}$)amide de formule (I).

**Revendications pour l'Etat contractant suivant : ES**

**1.** Procédé pour la séparation enzymatique de dérivés de l'acide PTC-(2-amino-4-méthylphospinobutyrique) caractérisé en ce qu'on traite un mélange de dérivés D- et L-PTC de formule générale I :

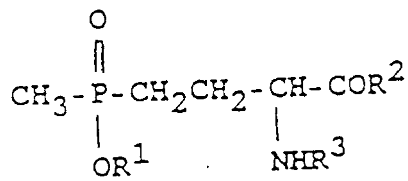

(I),

dans laquelle

a)

$R^1$ représente un alkyle en $C_1$-$C_{20}$ linéaire ou ramifié qui peut être non substitué ou substitué par un ou plusieurs radicaux halogène, ou une ou plusieurs fois par un alcoxy en $C_1$-$C_8$, ou représente un cycloalkyle en $C_3$-$C_8$, qui peut être substitué par un ou plusieurs groupes pris parmi les alkyle en $C_1$-$C_4$, alcoxy en $C_1$-$C_4$ et halogène, ou représente les alcènyle en $C_3$-$C_{10}$, alcynyle en $C_3$-$C_{10}$ ou benzyle,

$R^2$ représente les hydroxy, alcoxy en $C_1$-$C_{20}$ linéaire ou ramifié qui est non substitué ou substitué par un ou plusieurs radicaux halogène une ou plusieurs fois par un alcoxy en $C_1$-$C_8$ ou représente les amino ou (alkyle en $C_1$-$C_{20}$)amino et

$R^3$ représente les formyle, (alkyle en $C_1$-$C_{20}$)carbonyle linéaire ou ramifié, qui est non substitué ou substitué dans la partie alkyle par un ou plusieurs radicaux pris dans le groupe des hydroxy, halogène, phényle, lequel phényle peut être substitué par jusqu'à 3 radicaux pris parmi les groupes alkyle en $C_1$-$C_{12}$, alcoxy en $C_1$-$C_{12}$, halogène, nitro et $CF_3$, (alkyle en $C_1$-$C_{20}$)carbonyle est aussi substitué par alcoxy en $C_1$-$C_4$ et alkylthio en $C_1$-$C_4$, $R^3$ représente aussi les benzoyle ou benzoyle substitué par 1 à 3 radicaux pris dans le groupe comportant les alkyle en $C_1$-$C_{12}$, alcoxy en $C_1$-$C_{12}$, halogène, nitro et $CF_3$,

b)

$R^1$ est défini comme dans a),

$R^2$ est défini comme dans a), mais n'est pas l'hydroxy, et

$R^3$ est défini comme dans a) ou représente un autre groupe protecteur usuel de groupes amino,

en milieu aqueux ou aqueux-organique avec une enzyme à activité hydrolytique.

**2.** Procédé selon la revendication 1, caractérisé en ce qu'on utilise dans le cas a) une enzyme séparant les groupes N-acyle (acylase) et dans le cas b) une enzyme protéolytique ou à activité estérase en tant qu'enzyme à activité hydrolytique.

**3.** Procédé selon la revendication 1 ou 2, caractérisé en ce que $R^2$ ne signifie pas l'hydroxy et $R^3$ est défini comme dans la revendication 1 dans le point a) ou représente un autre groupe N-protecteur usuel, choisi parmi les (alcoxy en $C_1$-$C_{20}$)carbonyle et (alkyle en $C_1$-$C_{20}$)sulfonyle non ramifiés ou ramifiés, qui peuvent être chaque fois non substitués ou substitués dans la partie alkyle par un ou plusieurs radicaux pris dans le groupe comportant les hydroxy, halogène, alcoxy en $C_1$-$C_4$, alkylthio en $C_1$-$C_4$, phényle, phényle portant 1 à 3 substituants pris dans le groupe comportant les alkyles en $C_1$-$C_{12}$, alcoxy en $C_1$-$C_{12}$, halogène, nitro et $CF_3$, et représente un phénylsulfonyle, qui peut être substitué par jusqu'à trois radicaux pris dans le groupe des alkyes en $C_1$-$C_{12}$, alcoxy en $C_1$-$C_{12}$, halogène, nitro et $CF_3$.

**4.** Procédé selon la revendication 1 ou 2, caractérisé en ce qu'on utilise un mélange de dérivés de l'acide D- et L-PTC-(2-amino-4-méthylphosphinobutyrique) de formule (I) mentionnée selon la revendication 1, dans laquelle

a)

$R^1$ représente un alkyle en $C_1$-$C_{10}$ linéaire ou ramifié ou un alkyle en $C_1$-$C_{10}$ qui est substitué par des halogène ou alcoxy en $C_1$-$C_4$, ou représente un cycloalkyle en $C_5$-$C_6$,

$R^2$ représente les hydroxy, alcoxy en $C_1$-$C_8$ linéaire ou ramifié ou alcoxy en $C_1$-$C_8$ qui est une ou plusieurs fois substitué par des halogène ou alcoxy en $C_1$-$C_4$, ou représente les amino ou (alkyle en $C_1$-$C_{10}$)amino, et

$R^3$ représente les formyle, (alkyle en $C_1$-$C_{10}$)carbonyle linéaire ou ramifié, qui peut être non substitué ou substitué dans la partie alkyle par un ou deux radicaux pris dans le groupe

des hydroxy, halogène, phényle, un radical phényle qui est substitué par 1 à 3 radicaux pris parmi les groupes des alkyle en $C_1$-$C_4$, alcoxy en $C_1$-$C_4$ et halogène, et $R^3$ est substitué par les alcoxy en $C_1$-$C_4$ ou (alkyl en $C_1$-$C_4$)thio ou représente les benzoyle ou benzoyle qui est substitué par 1 à 3 radicaux pris parmi les alkyle en $C_1$-$C_4$, alcoxy en $C_1$-$C_4$, et halogène, ou

b)

$R^1$ est défini comme dans a),

$R^2$ est défini comme dans a), mais n'est pas l'hydroxy, et

$R^3$ est défini comme dans a) ou représente un (alcoxy en $C_1$-$C_{10}$)carbonyle qui est non substitué ou substitué par des hydroxy, halogène, méthoxy, éthoxy, phényle ou un radical phényle qui porte un à trois substituants pris dans le groupe des alkyle en $C_1$-$C_4$, alcoxy en $C_1$-$C_4$ et halogène.

**5.** Procédé selon la revendication 1 ou 2, caractérisé en ce qu'on utilise un mélange de dérivés de l'acide D- et L-PTC-(2-amino-4-méthylphosphinobutyrique) de formule (I) selon la revendication 1, dans laquelle

a)

$R^1$ représente un alkyle en $C_1$-$C_6$ linéaire ou ramifié ou le cyclohexyle,

$R^2$ représente les hydroxy, alcoxy en $C_1$-$C_6$ linéaire ou ramifié ou amino, et

$R^3$ représente un (alkyle en $C_1$-$C_4$)carbonyle ou un (alkyle en $C_1$-$C_4$)carbonyle, qui est substitué par un phényle ou par un phényle 1 à 3 fois substitué, dont les 1 à 3 substituants sont pris dans le groupe des alkyle en $C_1$-$C_4$, alcoxy en $C_1$-$C_4$ et halogène ou $R^3$ représente le benzoyle ou

b)

$R^1$ représente un alkyle en $C_1$-$C_6$ linéaire ou ramifié ou le cyclohexyle,

$R^2$ représente un alcoxy en $C_1$-$C_6$ linéaire ou ramifié, ou un amino, et

$R^3$ est défini comme dans le point a) ou représente un (alcoxy en $C_1$-$C_4$)-carbonyle linéaire ou ramifié, de préférence le tert-butyl-oxycarbonyle ou le benzyloxycarbonyle qui peut être substitué dans le cycle de phényle encore par un à trois radicaux pris dans le groupe comprenant les alkyle en $C_1$-$C_4$, alcoxy en $C_1$-$C_4$ et halogène.

**6.** Procédé selon la revendication 1 ou 2, caractérisé en ce que

a)

$R^1$ représente les méthyle, éthyle, propyle, butyle, pentyle, hexyle ou cyclohexyle,

$R^2$ représente les méthoxy, éthoxy, propoxy, butoxy, pentoxy, hexoxy ou $NH_2$, et

$R^3$ représente les acétyle ou phénacétyle ou

b)

$R^1$ est défini comme dans a),

$R^2$ est défini comme dans a), mais n'est pas l'hydroxy et

$R^3$ est défini comme dans a) ou représente les tert-butyl-oxycarbonyle ou benzyloxycarbonyle.

**7.** Procédé selon une ou plusieurs des revendications 1 à 6, caractérisé en ce qu'on utilise dans le cas

a) l'acylase I ou la pen-G-acylase, et dans le cas

b) les subtilisine, $\alpha$-chymotrypsine, papaine, ficine, bromélaine ou une autre enzyme de qualité industrielle en tant qu'enzyme à activité hydrolytique.

**8.** Procédé selon une ou plusieurs des revendications 1 à 7, caractérisé en ce qu'on effectue le clivage enzymatique à une température de 20 à 60 °C, de préférence de 20 à 50 °C.

**9.** Procédé selon une ou plusieurs des revendications 1 à 8, caractérisé en ce que, après le clivage enzymatique, les dérivés de l'acide D-PTC-(2-amino-4-méthylphosphinobutyrique) n'ayant pas réagi par voie enzymatique, sont éliminés par extraction avec un solvant organique.

**10.** Procédé selon une ou plusieurs des revendications 1 à 9, caractérisé en ce qu'on traite le dérivé obtenu de l'acide L-PTC(2-amino-4-méthylphosphinobutyrique) par une hydrolyse chimique et on isole le L-PTC.

**11.** Procédé selon une ou plusieurs des revendications 1 à 10, caractérisé en ce qu'on effectue le clivage enzymatique à un pH de 5 à 9, plus particulièrement de 6 à 8,5.

**12.** Procédé pour la préparation des composes de formule (I), définie selon la revendication 1, dans laquelle $R^1$ et $R^3$ sont définis selon une ou plusieurs des revendications 1 à 4 et $R^2$ représente un amino ou (alkyle en $C_1$-$C_{20}$) amino, caractérisé en ce que, un nitrile de formule

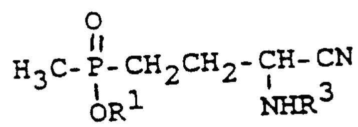

est hydrolysé en milieu acide sélectivement sur le groupe cyano et, si on le désire, l'amide de formule (I) obtenu est alkylé selon des méthodes usuelles en (alkyl en $C_1$-$C_{20}$)amide de formule (I).

**13.** Procédé selon la revendication 12, caractérisé en ce que $R^2$ représente un amino.

**14.** Procédé selon la revendication 13, caractérisé en ce que $R^1$ et $R^3$ sont définis selon la revendication 5 ou 6.

**15.** Procédé selon la revendication 14, caractérisé en ce que $R^1$ représente le cyclohexyle et $R^3$ le phénacétyle.